# EUROPÄISCHE PATENTSCHRIFT

(11) **EP 0 721 948 B1**
(45) Veröffentlichungstag und Bekanntmachung des Hinweises auf die Patenterteilung: **03.12.1997**
(21) Anmeldenummer: 96100031.2
(22) Anmeldetag: 03.01.1996
(51) Int. Cl.: C07D 401/04, A61K 31/47, C07D 471/04, C07D 513/04, C07D 209/44

(54) **Chinolon- und Naphthyridoncarbonsäure-Derivate als antibakterielle Mittel**
Quinolone- and naphthyridone carboxylic acid derivatives as antibacterial agents
Dérivés carboxyliques de quinolone et naphthyridone comme agents antibactériens

(30) Priorität: 13.01.1995 DE 19500792
(43) Veröffentlichungstag der Anmeldung: 17.07.1996
(73) Patentinhaber: BAYER AG, 51368 Leverkusen (DE)
(72) Erfinder: Petersen, Uwe, Dr., D-51375 Leverkusen (DE); Ruther, Michael, Dr., D-40789 Monheim (DE); Schenke, Thomas, Dr., D-51469 Bergisch Gladbach (DE); Bremm, Klaus Dieter, Dr., D-45661 Recklinghausen (DE); Endermann, Rainer, Dr., D-42113 Wuppertal (DE)

(56) Entgegenhaltungen:
- EP-A- 0 520 240
- EP-A- 0 550 019
- EP-A- 0 588 166

## Beschreibung

Die Erfindung betrifft neue Chinolon- und Naphthyridoncarbonsäure-Derivate, die in 7-Stellung durch einen ungesättigten bicylischen Aminrest substituiert sind, ihre Salze, Verfahren zu ihrer Herstellung sowie diese enthaltende antibakterielle Mittel.

Es sind bereits aus den Patentanmeldungen EP 520 240 (Bayer), DE 4 230 804 (Bayer) sowie JP 4 253 973 (Banyu) Chinoloncarbonsäuren bekannt, die in 7-Stellung durch einen bicyclischen ungesättigten Aminrest substituiert sind. Diese Verbindungen zeichnen sich durch eine hohe antibakterielle Aktivität aus. Sie besitzen aber den Nachteil, daß sie ein relativ hohes gentoxisches Potential aufweisen, was ihren Einsatz als Arzneimittel problematisch macht. Der Erfindung liegt daher die Aufgabe zugrunde, Verbindungen aufzufinden, die bei hoher antibakterieller Wirksamkeit eine Verringerung der gentoxischen Eigenschaften aufweisen.

Es wurde jetzt gefunden, daß die Verbindungen der Formel (I)

T-Q (I)

in welcher
- Q: einen Rest der Formeln 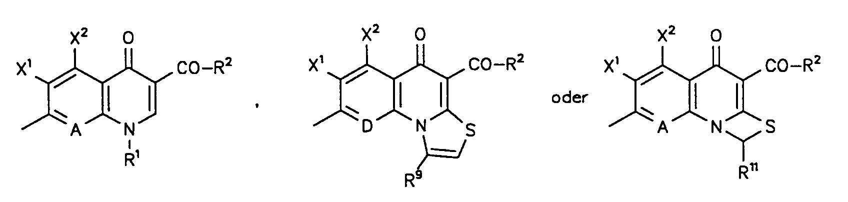 bedeutet, worin
- R¹: für gegebenenfalls durch Halogen oder Hydroxy ein- oder zweifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl,
- R²: für Hydoxy, gegebenenfalls durch Hydroxy, Methoxy, Amino, Dimethylamino substituiertes Alkoxy mit 1 bis 3 Kohlenstoffatomen, Benzyloxy oder (5-Methyl-2-oxo-1,3-dioxol-4-yl)-methyloxy, Acetoxymethyloxy, Pivaloyloxymethyloxy, 5-Indanyloxy, Phthalidinyloxy, 3-Acetoxy-2-oxo-butyloxy, Nitromethyl oder Dialkoxycarbonylmethyl mit 1 bis 2 Kohlenstoffatomen in jedem Alkylteil,
- R⁹: für Wasserstoff oder gegebenenfalls durch Methoxy, Hydroxy oder Halogen substituiertes Alkyl mit 1 bis 3 Kohlenstoffatomen,
- R¹¹: für Wasserstoff, CH₃, CH₂F oder =CH₂,
- X¹: für Wasserstoff, Halogen oder Nitro,
- X²: für Wasserstoff, Halogen, Amino, Hydroxy, Methoxy, Mercapto, Methyl, Halogenmethyl oder Vinyl,
- A: für H oder C-R⁷ steht, worin
- R⁷: für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*S-CH₂-CH-CH₃, -*CH₂-CH₂-CH-CH₃ oder -*O-CH₂-N-R⁸, wobei das mit * markierte Atom mit dem Kohlenstoffatom von A verknüpft ist und worin
- R⁸: Wasserstoff, Methyl oder Formyl bedeutet,
bilden kann, und
- D: für N oder C-R¹⁰ steht, worin
- R¹⁰: für Wasserstoff Halogen, CF₃, OCH₃, OCHF₂ oder CH₃ steht oder auch gemeinsam mit R⁹ eine Brücke der Struktur -*O-CH₂-, -*NH-CH₂-, -*N(CH₃)-CH₂-, -*N(C₂H₅)-CH₂-, -*N(C₃H₅)-CH₂- oder -*S-CH₂- bilden kann, wobei das mit * markierte Atom mit dem Kohlenstoffatom von D verknüpft ist, und
- T: einen Rest der Formel 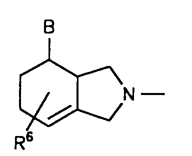
bedeutet, worin
- B: für (CH₂)ₘ-NR³R⁴ oder (CH₂)ₘ-OR⁵ steht, worin
- m: für 0 oder 1,
- R³: für Wasserstoff gegebenenfalls durch Hydroxy substituiertes Alkyl mit 1 bis 3 Kohlenstoffatomen, Acyl mit 1 bis 3 Kohlenstoffatomen oder Alkoxycarbonyl mit 1 bis 4 Kohlenstoffatomen im Alkylteil,
- R⁴: für Wasserstoff oder Methyl und
- R⁵: für Wasserstoff oder Methyl und
- R⁶: für Wasserstoff oder Methyl steht,
und deren pharmazeutisch verwendbaren Hydrate und Säureadditionssalze sowie die Alkali-, Erdalkali-, Silber- und Guanidiniumsalze der zugrundeliegenden Carbonsäuren bei guter Verträglichkeit eine hohe antibakterielle Wirkung insbesondere gegenüber grampositiven Bakterien aufweisen.

Bevorzugt sind die Verbindungen der Formel (I),
in welcher
- Q: einen Rest der Formeln 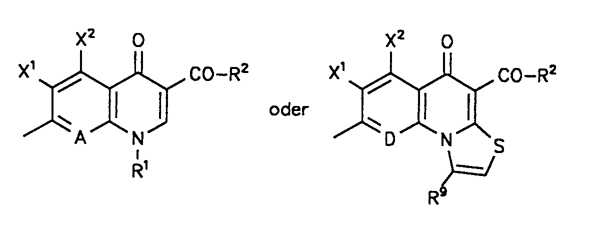
bedeutet,worin
- R¹: für gegebenenfalls durch Halogen ein- oder zweifach substituiertes Alkyl mit 1 bis 4 Kohlenstoffatomen, Alkenyl mit 2 bis 3 Kohlenstoffatomen, gegebenenfalls durch 1 Fluoratom substituiertes Cycloalkyl mit 3 oder 4 Kohlenstoffatomen, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl,
- R²: für Hydroxy, Alkoxy mit 1 bis 2 Kohlenstoffatomen, Benzyloxy oder (5-Methyl-2-oxo-1,3-dioxol-4-yl)-methyloxy,
- R⁹: für Wasserstoff oder gegebenenfalls durch Fluor ein- bis dreifach substituiertes Alkyl mit 1 bis 2 Kohlenstoffatomen,
- X¹: für Fluor oder Chlor,
- X²: für Wasserstoff, Halogen, Amino, Methyl, Trifluormethyl oder Vinyl,
- A: für N oder C-R⁷ steht, worin
- R⁷: für Wasserstoff Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*CH₂-CH₂-CH-CH₃ oder -*O-CH₂-N-R⁸, wobei das mit * markierte Atom mit dem Kohlenstoffatom von A verknüpft ist und worin
- R⁸: Wasserstoff oder Methyl bedeutet, bilden kann, und
- D: für N oder C-R¹⁰ steht, worin
- R¹⁰: für Wasserstoff Fluor, Chlor, CF₃, OCH₃ oder CH₃ steht oder auch gemeinsam mit R⁹ eine Brücke der Struktur -O-CH₂-, -*N(CH₃)-CH₂-, -*N(C₂H₅)-CH₂-, -*N(C₃H₅)-CH₂- oder -*S-CH₂- bilden kann, wobei das mit * markierte Atom mit dem Kohlenstoffatom von D verknüpft ist, und
- T: einen Rest der Formel 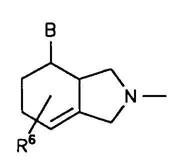
bedeutet, worin
- B: für -NR³R⁴ oder -OH steht, worin
- R³: für Wasserstoff oder Methyl,
- R⁴: für Wasserstoff oder Methyl und
- R⁶: für Wasserstoff steht
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I), in welcher
- Q: einen Rest der Formel 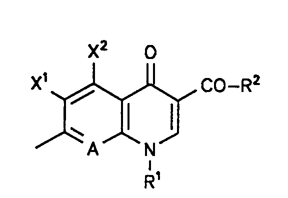
bedeutet, worin
- R¹: für gegebenenfalls durch Fluor ein- oder zweifach substituiertes Alkyl mit 1 bis 4 Kohlenstoffatomen, Vinyl, gegebenenfalls durch 1 Fluoratom substituiertes Cyclopropyl, gegebenenfalls durch Fluor ein- oder zweifach substituiertes Phenyl,
- R²: für Hydroxy, Alkoxy mit 1 bis 2 Kohlenstoffatomen oder (5-Methyl-2-oxo-1,3-dioxol-4-yl)-methyloxy,
- X¹: für Fluor,
- X²: für Wasserstoff, Fluor, Amino, Methyl oder Vinyl,
- A: für N oder C-R⁷ steht, worin
- R⁷: für Wasserstoff, Fluor, Chlor, Brom, CF₃, OCH₃, OCHF₂, CH₃, CN, CH=CH₂ oder C≡CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin
- R⁸: Wasserstoff oder Methyl bedeutet, bilden kann, und
- T: einen Rest der Formel 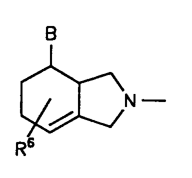
bedeutet, worin
- B: für NH₂ und
- R⁶: für Wasserstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man Verbindungen der Formel (II)

Y-Q (II) ,

in welcher
- Q: die oben angegebene Bedeutung hat und
- Y: für Halogen, inbesondere für Fluor oder Chlor steht,
mit Verbindungen der Formel (III) 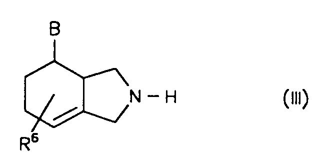 in welcher
- B und R⁶: die oben angegebenen Bedeutungen haben,
gegebenenfalls in Gegenwart von Säurefängern umsetzt und gegebenenfalls vorhandene Schutzgruppen abspaltet.

Verwendet man beispielsweise 6,7-Difluor-1-cyclopropyl-1,4-dihydro-8-methoxy-4-oxo-3-chinolincarbonsäure und (3aRS, 4RS)-2,3,3a,4,5,6-Hexahydro-1H-isoindol-4-ylamin als Ausgangsstoffe, so kann der Reaktionsverlauf durch das folgende Formelschema wiedergegeben werden:

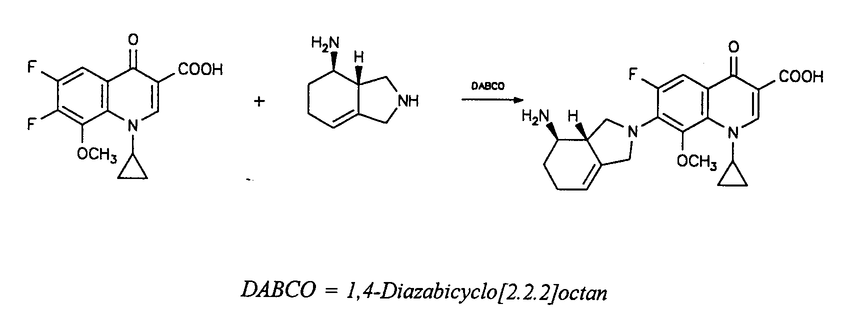

Die als Ausgangsverbindungen verwendeten Verbindungen der Formel (II) sind bekannt oder können nach bekannten Methoden hergestellt werden. Sie können gegebenenfalls sowohl als racemische als auch als enantiomerenreine Verbindungen eingesetzt werden. Als Beispiele seien genannt:

7-Chlor-1-cyclopropyl-6-fluor-1,4-dihydro-4-oxo-3-chinolincarbonsäure, 1-Cyclopropyl-6,7-difluor-1,4-dihydro-4-oxo-3-chinolincarbonsäure, 6-Chlor-1-cyclopropyl-7,8-difluor-1,4-dihydro-4-oxo-3-chinolincarbonsäure, 8-Chlor-1-cyclopropyl-6,7-difluor-1,4-dihydro-4-oxo-3-chinolincarbonsäure, 1-Cyclopropyl-6,7,8-trifluor-1,4-dihydro-4-oxo-3-chinolincarbonsäure, 5-Brom-1-cyclopropyl-6,7,8-trifluor-1,4-dihydro-4-oxo-3-chinolincarbonsäure, 5-Brom-1-(2,4-difluorphenyl)-6,7,8-trifluor-1,4-dihydro-4-oxo-3-chinolincarbonsäure, 1-Cyclopropyl-6,7-difluor-1,4-dihydro-8-methyl-4-oxo-3-chinolincarbonsäure, 6,7-Difluor-1-ethyl-1,4-dihydro-4-oxo-3-chinolincarbonsäure, 7-Chlor-6-fluor-1-ethyl-1,4-dihydro-4-oxo-3-chinolincarbonsäure, 7-Chlor-6-fluor-1,4-dihydro-1-(2-hydroxyethyl)-4-oxo-3-chinolincarbonsäure, 6,7-Difluor-1-(2-fluorethyl)-1,4-dihydro-4-oxo-3-chinolincarbonsäure, 7-Chlor-6-fluor-1,4-dihydro-1-methoxy-4-oxo-3-chinolincarbonsäure, 7-Chlor-6-fluor-1,4-dihydro-1-methylamino-4-oxo-3-chinolincarbonsäure, 6,7-Difluor-1,4-dihydro-4-oxo-1-phenyl-3-chinolincarbonsäure, 7-Chlor-1-cyclopropyl-6-fluor-1,4-dihydro-4-oxo-1,8-naphthyridin-3-carbonsäure, 7-Chlor-1-cyclopropyl-6-fluor-1,4-dihydro-4-oxo-1,8-naphthyridin-3-carbonsäure-ethylester, 1-Cyclopropyl-6,7,8-trifluor-1,4-dihydro-4-oxo-3-chinolincarbonsäureethylester, 9,10-Difluor-2,3-dihydro-3-methyl-7-oxo-7H-pyrido-[1,2,3-de][1,4]benzoxacin-6-carbonsäure, 8,9-Difluor-6,7-dihydro-5-methyl-1-oxo-1H,5H-benzo[i,j]-chinolicin-2-carbonsäure, 7-Chlor-6-fluor-1-phenyl-1,4-dihydro-4-oxo-1,8-naphthyridin-3-carbonsäure, 7-Chlor-6-fluor-1-(4-fluorphenyl)-1,4-dihydro-4-oxo-1,8-naphthyridin-3-carbonsäureethylester,
6,7,8-Trifluor-1,4-dihydro-1-methylamino-4-oxo-3-chinolincarbonsäure, 1-Amino-6,7,8-trifluor-1,4-dihydro-4-oxo-3-chinolincarbonsäure, 6,7,8-Trifluor-1,4-dihydro-1-dimethylamino-4-oxo-3-chinolincarbonsäure, 6,7-Difluor-1-(4-fluorphenyl)-1,4-dihydro-8-methyl-4-oxo-3-chinolincarbonsäure, 7-Chlor-6-fluor-1-(4-fluorphenyl)-1,4-dihydro-4-oxo-3-chinolincarbonsäure, 7-Chlor-6-fluor-1-(2,4-difluorphenyl)-1,4-dihydro-4-oxo-3-chinolincarbonsäure, 6,7,8-Trifluor-1-(4-fluorphenyl)-1,4-dihydro-4-oxo-3-chinolincarbonsäure, 1-Cyclopropyl-6,7-difluor-1,4-dihydro-5-methyl-4-oxo-3-chinolincarbonsäure, 7-Chlor-1-cyclopropyl-6-fluor-1,4-dihydro-5-methyl-4-oxo-1,8-naphthyridin-3-carbonsäure, 7-Chlor-6-fluor-1-(2,4-difluorphenyl)-1,4-dihydro-5-methyl-4-oxo-1,8-naphthyridin-3-carbonsäure, 6,7-Difluor-1,4-dihydro-1-(3-oxetanyl)-4-oxo-3-chinolincarbonsäure, 6,7,8-Trifluor-1,4-dihydro-1-(3-oxetanyl)-4-oxo-3-chinolincarbonsäure, 1-(Bicyclo[1.1.1]pent-1-yl)-6,7,8-trifluor-1,4-dihydro-4-oxo-3-chinolincarbonsäure, 7-Chlor-1-(1,1-dimethylpropargyl)-6-fluor-1,4-dihydro-4-oxo-1,8-naphthyridin-3-carbonsäure, 6,7,8-Trifluor-1-(2,4-difluorphenyl)-1,4-dihydro-4-oxo-3-chinolincarbonsäure, 6,7,8-Trifluor-1,4-dihydro-4-oxo-1-phenyl-3-chinolincarbonsäure, 7-Chlor-1-ethyl-6-fluor-1,4-dihydro-4-oxo-1,8-naphthyridin-3-carbonsäure, 6,7-Difluor-1,4-dihydro-4-oxo-1-vinyl-3-chinolincarbonsäure, 1-Cyclopropyl-5,6,7,8-tetrafluor-1,4-dihydro-4-oxo-3-chinolincarbonsäure, 5-Amino-1-cyclopropyl-6,7,8-trifluor-1,4-dihydro-4-oxo-3-chinolincarbonsäure, 1-Cyclopropyl-6,7,8-trifluor-1,4-dihydro-5-hydroxy-4-oxo-3-chinolincarbonsäure, 1-Cyclopropyl-6,7-difluor-1,4-dihydro-8-methoxy-4-oxo-3-chinolincarbonsäure, 7-Chlor-1-(2,4-difluorphenyl)-6-fluor-1,4-dihydro-4-oxo-1,8-naphthyridin-3-carbonsäureethylester, 1-Cyclopropyl-6,7-difluor-1,4-dihydro-4-oxo-8-vinyl-3-chinolincarbonsäure, 1-Cyclopropyl-8-ethinyl-6,7-difluor-1,4-dihydro-4-oxo-3-chinolincarbonsäure, 9,10-Difluor-3-methyl-7-oxo-2,3-dihydro-7H-pyrido[1,2,3-d,e][1,3,4]benzoxadiazin-6-carbonsäure, 8-Amino-9,10-difluor-3-methyl-7-oxo-2,3-dihydro-7H-pyrido[1,2,3-d,e][1,3,4]benzoxadiazin-6-carbonsäure, 7,8-Difluor-5-oxo-9,1-[(N-methylimino)methano]-5H-thiazolo[3,2-a]-chinolin-4-carbonsäure, 7,8-Difluor-5-oxo-9,1-[(N-ethylimino)methano]-5H-thiazolo[3,2-a]chinolin-4-carbonsäure, 7,8-Difluor-5-oxo-9,1-(epoxymethano)-5H-thiazolo[3,2-a]-chinolin-4-carbonsäure, 7,8-Difluor-5-oxo-9,1-(epithiomethano)-5H-thiazolo[3,2-a]-chinolin-4-carbonsäure, 7,8-Difluor-1-methyl-5-oxo-5H-thiazolo[3,2-a]-chinolin-4-carbonsäure, 8-Brom-6,7-difluor-1-(cis-2-fluorcyclopropyl)-1,4-dihydro-4-oxo-3-chinolincarbonsäure, 8-Chlor-6,7-difluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-4-oxo-3-chinolincarbonsäure, 6,7,8-Trifluor-1-(cis-2-fluorcyclopropyl)-1,4-dihydro-4-oxo-3-chinolincarbonsäure, 5,6,7,8-Tetrafluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-8-methyl-4-oxo-3-chinolincarbonsäure, 8-Ethinyl-6,7-difluor-1-(cis-2-fluorcyclopropyl)-1,4-dihydro-4-oxo-3-chinolincarbonsäure, 6,7-Difluor-1-(cis-2-fluorcyclopropyl)-1,4-dihydro-4-oxo-8-trifluormethyl-3-chinolincarbonsäure, 6,7-Difluor-1-(cis-2-fluorcyclopropyl)-8-difluormethoxy-1,4-dihydro-4-oxo-3-chinolincarbonsäure, 6,7-Difluor-1-(cis-2-fluorcyclopropyl)-1,4-dihydro-8-methoxy-4-oxo-3-chinolincarbonsäure, 6,7-Difluor-1-(cis-2-fluorcyclopropyl)-1,4-dihydro-5-methyl-4-oxo-3-chinolincarbonsäure, 5-Amino-6,7,8-trifluor-1-(cis-2-fluorcyclopropyl)-1,4-dihydro-4-oxo-3-chinolincarbonsäure, 8-Brom-6,7-difluor-1-[(1R,2S)-2-fluorcyclopropyl]-1,4-dihydro-4-oxo-3-chinolincarbonsäure, 8-Chlor-6,7-difluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-4-oxo-3-chinolincarbonsäure, 6,7,8-Trifluor-1-[(1R,2S)-2-fluorcyclopropyl]-1,4-dihydro-4-oxo-3-chinolincarbonsäure, 5,6,7,8-Tetrafluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-8-methyl-4-oxo-3-chinolincarbonsäure, 8-Ethinyl-6,7-difluor-1-[(1R,2S)-2-fluorcyclopropyl]-1,4-dihydro-4-oxo-3-chinolincarbonsäure, 6,7-Difluor-1-[(1R,2S)-2-fluorcyclopropyl]-1,4-dihydro-4-oxo-8-trifluormethyl-3-chinolincarbonsäure, 6,7-Difluor-1-[(1R,2S)-2-fluorcyclopropyl]-8-difluormethoxy-1,4-dihydro-4-oxo-3-chinolincarbonsäure, 6,7-Difluor-1-[(1R,2S)-2-fluorcyclopropyl]-1,4-dihydro-8-methoxy-4-oxo-3-chinolincarbonsäure, 6,7-Difluor-1-[(1R,2S)-2-fluorcyclopropyl]-1,4-dihydro-5-methyl-4-oxo-3-chinolincarbonsäure, 5-Amino-6,7,8-Trifluor-1-[(1R,2S)-2-fluorcyclopropyl]-1,4-dihydro-4-oxo-3-chinolincarbonsäure, 6,7-Difluor-4-oxo-4H-[1,3]thiazeto[3,2-a]chinolin-3-carbonsäure, 6,7-Difluor-1-methyl-4-oxo-4H-[1,3]thiazeto[3,2-a]chinolin-3-carbonsäure, 6,7-Difluor-1-fluormethyl-4-oxo-4H-[1,3]thiazeto[3,2-a]chinolin-3-carbonsäure.

Die als Ausgangsstoffe verwendeten Verbindungen der Formel (III) sind neu. Sie können dadurch erhalten werden, daß man von einer Verbindung der Formel (1), 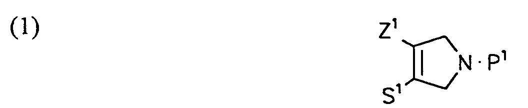 in welcher Z¹ beispielsweise Alkoxycarbonyl, P¹ eine geeignete Schutzgruppe am Stickstoff beispielsweise Benzyl, Phenylethyl oder Alkoxycarbonyl mit 1 bis 4 Kohlenstoffatomen im Alkylteil und S¹ Wasserstoff oder Methyl bedeutet, ausgeht, und diese durch gängige Reduktionsverfahren in Allylalkohole der Formel (2) 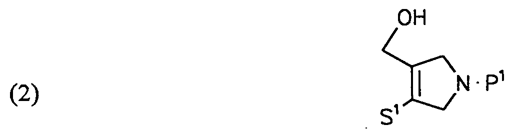 überführt, und diese unter Verwendung geeigneter Oxidationsmittel, wie zum Beispiel Pyridinchlorochromat oder DMSO/Oxalylchlorid/Triethylamin (Swern-Oxydation), zur Zwischenstufe der Formel (3) 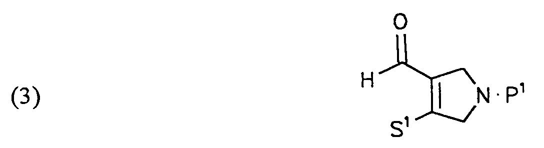 umsetzt, welche man in einer Wittig-Reaktion oder vergleichbaren Reaktionen zu einer Verbindung der Formel (4), 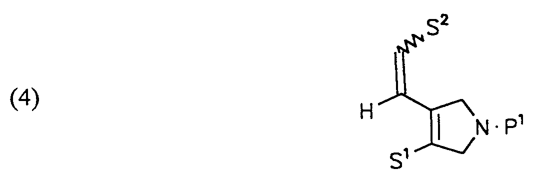 in welcher S² Wasserstoff oder Methyl bedeutet, überführt, diese mit einer Verbindung der Formel (5) 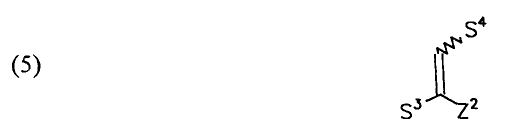 in welcher Z² beispielsweise Cyano, Alkoxycarbonyl, Aryloxycarbonyl oder Nitro und S³ und S⁴ gleich oder verschieden sind und für Wasserstoff oder Methyl stehen, in einer Cycloaddition zu Verbindungen der Formel (6), die im folgenden in einer vereinfachten Schreibweise formuliert werden, 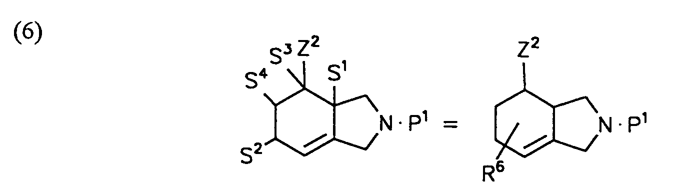 umsetzt. Aus dem anfallenden Reaktionsgemisch wird durch geeignete Trennmethoden beispielsweise die Verbindung der Formel (7) 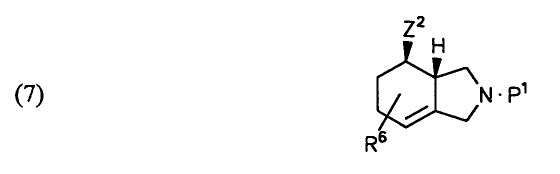 abgetrennt, welche im Falle P¹ = Benzyl oder α-Phenylethyl durch Umsetzung mit Chlorameisensäureestern oder Phosgen/Alkohol zu einer Verbindung der Formel (8), 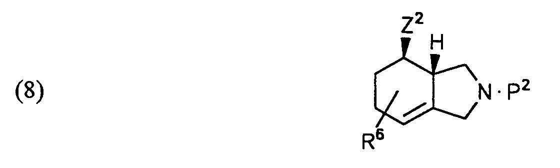 in welcher P² für Alkoxycarbonyl mit 1 bis 4 Kohlenstoffatomen im Alkylteil steht, umsetzt, welche durch geeignete saure oder basische Hydrolysebedingungen zu einer Säure der Formel (9) 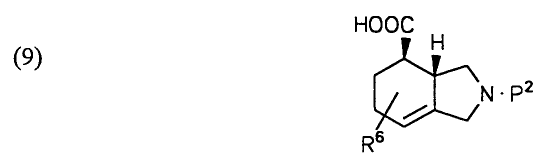 umgesetzt wird, welche beispielsweise durch einen Curtius-Abbau über ein intermediär entstehendes Azid der Formel (10) 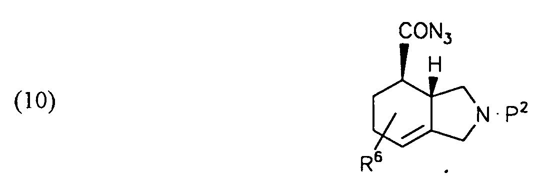 zu einem Isocyanat der Formel (11) 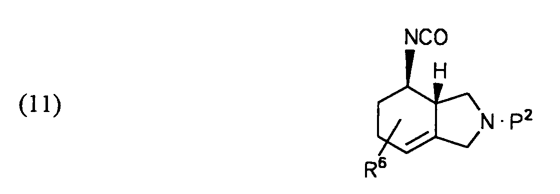 umgesetzt wird, das durch Alkoholyse zu einer Verbindung der Formel (12), 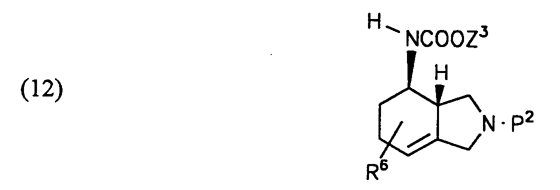 in der Z³ für C₁-C₄-Alkyl steht, umgesetzt wird, welche gegebenenfalls mit einem Alkylierungsmittel, beispielsweise mit Methyliodid, zu einer Verbindung der Formel (13), 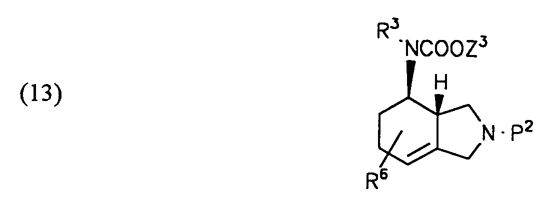 in der R³ Wasserstoff oder Alkyl bedeutet, überführt und durch Hydrolyse mit geeigneten Säuren oder Basen zu einer Verbindung der Formel (14) 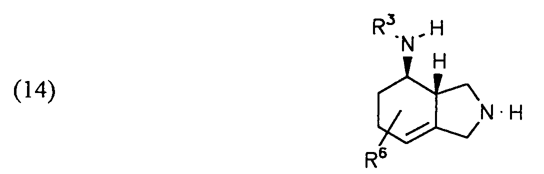 überführt. Verbindungen der Formel (14) entsprechen der allgemeinen Formel (III). Zu Verbindungen der Formel (III), in welcher B für Dialkylamino steht, gelangt man ausgehend von einer Verbindung (13), wenn man die Gruppe COOZ³, die beispielsweise eine tert-Butoxycarbonylschutzgruppe bedeuten kann, selektiv abspaltet und die freiwerdende Stickstofffunktion anschließend alkyliert.

Man kann im Anschluß an die Diels-Alder-Reaktion, die zu den Verbindungen der Formel (6) führt, auch die isomeren Verbindungen mit umgekehrter Konfiguration am Brückenkopfkohlenstoffatom isolieren, die sich in entsprechender Weise, wie es bereits für die Verbindungsreihe (6) bis (14) ausgeführt wurde, zu Verbindungen der Formel (15) 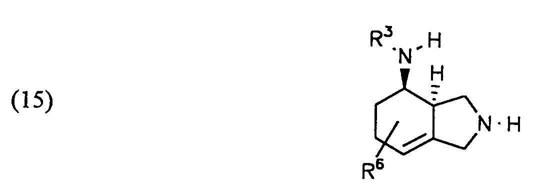 umsetzen lassen. Verbindungen der Formel (15) entsprechen der allgemeinen Formel (III).

Zu Verbindungen der Formel (III), in welcher B für Hydroxymethyl steht, gelangt man, wenn man eine Verbindung der Formel (7), in welcher Z² für Alkoxycarbonyl steht, beispielsweise mit komplexen Hydriden wie LiAlH₄ reduziert.

Zu Verbindungen der Formel (III), in welcher B für Aminomethyl steht, gelangt man, wenn man eine Cyanogruppe in einer Verbindung der Formel (7) beispielsweise über eine Reduktion mit komplexen Hydriden wie LiAlH₄ in eine Aminomethylgruppe umwandelt, die gegebenenfalls noch alkyliert werden kann. Daran schließt sich die Abspaltung der Schutzgruppe P¹ an.

Als Beispiele für ungesättigte bicyclische Amine der Formel (III), die sowohl als diastereomerenreine oder enantiomerenreine Verbindungen als auch als Diastereomeren- oder Enantiomerengemische vorliegen können, seien genannt:

2,3,3a,4,5,6-Hexahydro-1H-isoindol-4-ylamin, 4-Methyl-2,3,3a,4,5,6-hexahydro-1H-isoindol-4-ylamin, 5-Methyl-2,3,3a,4,5,6-hexahydro-1H-isoindol-4-ylamin, 6-Methyl-2,3,3a,4,5,6-hexahydro-1H-isoindol-4-ylamin, 7-Methyl-2,3,3a,4,5,6-hexahydro-1H-isoindol-4-ylamin, 4-Methylamino-2,3,3a,4,5,6-hexahydro-1H-isoindol, 4-Dimethylamino-2,3,3a,4,5,6-hexahydro-1H-isoindol, 4-Aminomethyl-2,3,3a,4,5,6-hexahydro-1H-isoindol, 4-Methylaminomethyl-2,3,3a,4,5,6-hexahydro-1H-isoindol, 4-Hydroxy-2,3,3a,4,5,6-hexahydro-1H-isoindol, 4-Hydroxymethyl-2,3,3a,4,5,6-hexahydro-1H-isoindol.

Die enantiomerenreinen Ausgangsverbindungen der Formel (III) können nach folgenden Verfahren hergestellt werden:
1. Die racemischen bicyclischen Amine (III) können mit enantiomerenreinen Säuren, zum Beispiel Carbonsäuren oder Sulfonsäuren wie N-Acetyl-L-glutaminsäure, N-Benzoyl-L-alanin, 3-Brom-campher-9-sulfonsäure, Campher-3-carbonsäure, cis-Camphersäure, Campher-10-sulfonsäure, O,O'-Dibenzoyl-weinsäure, D- oder L-Weinsäure, Mandelsäure, α-Methoxy-phenylessigsäure, 1-Phenyl-ethansulfonsäure, α-Phenyl-bernsteinsäure zu einem Gemisch der diastereomeren Salze umgesetzt werden, die sich durch fraktionierte Kristallisation zu den diastereomerenreinen Salzen trennen lassen (siehe P. Newman, Optical Resolution Procedures for Chemical Compounds, Volume 1). Durch Behandlung dieser Salze mit Alkali- oder Erdalkalihydroxyden lassen sich die enantiomerenreinen Amine freisetzen.
2. In ähnlicher Weise wie unter 1. beschrieben läßt sich eine Racematspaltung der basischen Zwischenstufen, die bei der Herstellung der racemischen bicyclischen Amine auftreten, mit den oben aufgeführten enantiomerenreinen Säuren durchführen.
3. Sowohl die racemischen Amine (III) als auch einige der zu (III) führenden Zwischenstufen können, gegebenenfalls nach Acylierung, über chirale Trägermaterialien chromatographisch getrennt werden (siehe zum Beispiel G. Blaschke, Angew. Chem. **92**, 14 [1980]).
4. Die racemischen Amine (III) können auch durch chemische Verknüpfung mit chiralen Acylresten in Diastereomerengemische überführt werden, die sich durch Destillation, Kristallisation oder Chromatographie in die diastereomerenreinen Acylderivate trennen lassen, aus denen sich durch Verseifung die enantiomerenreinen Amine isolieren lassen. Beispiele für Reagentien zur Verknüpfung mit chiralen Acylresten sind: α-Methoxy-α-trifluormethyl-phenyl-acetylchlorid, Menthylisocyanat, D- oder L-α-Phenyl-ethyl-isocyanat, Chlorameisensäure-menthylester, Campher-10-sulfonsäurechlorid.
5. Im Verlauf der Synthese der bicyclischen Amine (III) können statt achiraler auch chirale Schutzgruppen eingeführt werden. Man gelangt auf diese Weise zu Diastereomerengemischen, die sich auftrennen lassen. Zum Beispiel kann bei der Synthese der Zwischenstufe (7) ein gegebenenfalls vorhandener α-Phenylethylrest in der R- oder S-Konfiguration vorliegen.

Die Umsetzung von (II) mit (III), bei der die Verbindungen (III) auch in Form ihrer Salze, wie zum Beispiel der Hydrochloride, eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 160°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 5 Mol der Verbindung (III) ein.

Freie Aminogruppen können während der Umsetzung durch eine geeignete Aminoschutzgruppe, wie zum Beispiel durch den tert.-Butoxycarbonylrest oder eine Azomethin-Schutzgruppe, geschützt und nach Beendigung der Reaktion wieder freigesetzt werden.

Die Herstellung von erfindungsgemäßen Verbindungen der Formel (I), in welcher R² für CH₂NO₂ oder Dialkoxycarbonylmethyl steht, kann auch durchgeführt werden, indem man eine Verbindung der Formel (I), in welcher R² für OH steht, mit einem Aktivierungsmittel wie Carbonyldiimidazol in einem Lösungsmittel wie Tetrahydrofuran, Dichlormethan oder Chloroform umsetzt und anschließend mit einer CH-aciden Verbindung wie Nitromethan oder Malonsäuredialkylester umsetzt. Diese Umsetzung wird bevorzugt in einem Lösungsmittel wie Tetrahydrofuran in Gegenwart einer Base (Natriumhydrid, Kaliumcarbonat, Natriumcarbonat) durchgeführt.

Die Herstellung von erfindungsgemäßen Verbindungen der Formel (I), in welcher X² = NH₂ bedeutet, gelingt auch durch Umsetzung von Verbindungen der Formel (I), in welcher X² = F bedeutet, mit Ammoniak in polaren Lösungsmitteln wie Dimethylsulfoxid bei Temperaturen von 50°C bis 120°C bei Normaldruck oder durch Erhitzen im Autoklaven. Die Herstellung von erfindungsgemäßen Verbindungen der Formel (I), in welcher A = C-OCH₃ bedeutet, gelingt auch durch Umsetzung von Verbindungen der Formel (I), in welcher A = C-F bedeutet, mit Alkalimethylaten, wie zum Beispiel Natriummethylat oder Kaliummethylat, in Lösungsmitteln wie zum Beispiel Dimethylformamid, Glykoldimethylether, Dioxan, Tetrahydrofuran, Dimethylsulfoxid, Hexamethylphosphorsäuretrisamid oder Alkoholen bei Temperaturen von 20°C bis 150°C. Die Umsetzung kann bei Verwendung tiefsiedender Lösungsmittel auch im Autoklaven unter Druck durchgeführt werden. Durch Zugabe von Kronenethern, wie zum Beispiel 15-Crown-5 oder 18-Crown-6 läßt sich die Reaktion beschleunigen und bei niedrigerer Temperatur durchführen.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20 bis 180°C, vorzugsweise etwa 60 bis 120°C, umgesetzt. Das entstehende Reaktionswasser kann auch durch azeotrope Destillation mit Chloroform, Tetrachlormethan oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten Ester, wie zum Beispiel (5-Methyl-2-oxo-1,3-dioxol-4-yl-methyl)-ester, werden durch Umsetzung eines Alkalisalzes der zugrundliegenden Carbonsäure, die gegebenenfalls am N-Atom durch eine Schutzgruppe geschützt sein kann, mit 4-Brommethyl- oder 4-Chlormethyl-5-methyl-1,3-dioxol-2-on in einem Lösungsmittel wie Dimethylformamid, Dimethylacetamid, N-Methyl-pyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen in überschüssiger wäßriger Säure und Ausfällen des Salzes mit einem mit Wasser mischbaren Lösungsmittel wie Methanol, Ethanol, Aceton oder Acetonitril. Man kann auch äquivalente Menge Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, 2-Hydroxyglutarsäure, Methansulfonsäure, 4-Toluolsulfonsäure, Galacturonsäure, Glucuronsäure, 5-Oxotetrahydrofuran-2-carbonsäure, Embonsäure, Glutaminsäure oder Asparaginsäure zu verstehen.

Die Alkali- und Erdalkalisalze der erfindungsgemäßen Carbonsäuren werden beispielsweise durch Lösen des Betains in überschüssiger Alkali- oder Erdalkalilauge, Filtration vom ungelösten Betain und Eindampfen bis zur Trockne erhalten. Pharmazeutisch geeignet sind Natrium, Kalium- und Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die nachfolgend aufgeführten sowie die in den folgenden Tabellen aufgeführten Wirkstoffe hergestellt werden, die sowohl als Racemate oder als enantiomerenreine Verbindungen oder gegebenenfalls auch als Diastereomerengemische oder als diastereomerenreine Verbindungen vorliegen können:

8-[(3aRS,4RS)-4-Amino-2,3,3a,4,5,6-hexahydro-1H-isoindol-2-yl]-7-fluor-5-oxo-9,1-(epoxymethano)-5H-thiazolo[3,2-a]chinolin-4-carbonsäure, 7-Fluor-8-[(3aRS,4RS)-4-methylamino-2,3,3a,4,5,6-hexahydro-1H-isoindol-2-yl]-5-oxo-9,1-(epoxymethano)-5H-thiazolo[3,2-a]chinolin-4-carbonsäure, 8-(4-Aminomethyl-2,3,3a,4,5,6-hexahydro-1H-isoindol-2-yl)-7-fluor-5-oxo-9,1-(epoxymethano)-5H-thiazolo[3,2-a]chinolin-4-carbonsäure, 7-Fluor-8-(4-methylaminomethyl-2,3,3a,4,5,6-hexahydro-1H-isoindol-2-yl)-5-oxo-9,1-(epoxymethano)-5H-thiazolo[3,2-a]chinolin-4-carbonsäure, 8-[(3aRS,4RS)-4-Amino-2,3,3a,4,5,6-hexahydro-1H-isoindol-2-yl]-7-fluor-5-oxo-9,1-[(N-methyl-imino)methano]-5H-thiazolo[3,2-a]chinolin-4-carbonsäure, 7-Fluor-8-[(3aRS,4RS)-4-methylamino-2,3,3a,4,5,6-hexahydro-1H-isoindol-2-yl]-5-oxo-9,1-[(N-methyl-imino)methano]-5H-thiazolo[3,2-a]chinolin-4-carbonsäure, 8-(4-Aminomethyl-2,3,3a,4,5,6-hexahydro-1H-isoindol-2-yl)-7-fluor-5-oxo-9,1-[(N-methyl-imino)methano]-5H-thiazolo[3,2-a]chinolin-4-carbonsäure, 7-Fluor-8-(4-methylaminomethyl-2,3,3a,4,5,6-hexahydro-1H-isoindol-2-yl)-5-oxo-9,1-[(N-methyl-imino)methano]-5H-thiazolo[3,2-a]chinolin-4-carbonsäure, 10-[(3aRS,4RS)-4-Amino-2,3,3a,4,5,6-hexahydro-1H-isoindol-2-yl]-9-fluor-3-methyl-7-oxo-2,3-dihydro-7H-pyrido[1,2,3-d,e][1,3,4]benzoxadiazin-6-carbonsäure, 9-Fluor-3-methyl-10-[(3aRS,4RS)-4-methylamino-2,3,3a,4,5,6-hexahydro-1H-isoindol-2-yl]-7-oxo-2,3-dihydro-7H-pyrido[1,2,3-d,e][1,3,4]benzoxadiazin-6-carbonsäure, 10-[(3aRS,4RS)-4-Amino-2,3,3a,4,5,6-hexahydro-1H-isoindol-2-yl]-9-fluor-3-methyl-7-oxo-2,3-dihydro-7H-pyrido[1,2,3-d,e][1,3,4]benzoxadiazin-6-carbonsäure, 8-Amino-10-[(3aRS,4RS)-4-Amino-2,3,3a,4,5,6-hexahydro-1H-isoindol-2-yl]-9-fluor-3-methyl-7-oxo-2,3-dihydro-7H-pyrido[1,2,3-d,e][1,3,4]benzoxadiazin-6-carbonsäure, 10-(4-Dimethylaminomethyl-2,3,3a,4,5,6-hexahydro-1H-isoindol-2-yl)-9-fluor-3-methyl-7-oxo-2,3-dihydro-7H-pyrido[1,2,3-d,e][1,3,4]benzoxadiazin-6-carbonsäure, 9-Fluor-3-methyl-10-[(3aRS,4RS)-4-methylamino-2,3,3a,4,5,6-hexahydro-1H-isoindol-2-yl]-7-oxo-2,3-dihydro-7H-pyrido[1,2,3-d,e][1,4]benzoxazin-6-carbonsäure, 10-(4-Aminomethyl-2,3,3a,4,5,6-hexahydro-1H-isoindol-2-yl)-9-fluor-3-methyl-7-oxo-2,3-dihydro-7H-pyrido[1,2,3-d,e][1,4]benzoxazin-6-carbonsäure, 8-Amino-10-[(3aRS,4RS)-4-amino-2,3,3a,4,5,6-hexahydro-1H-isoindol-2-yl]-9-fluor-3-methyl-7-oxo-2,3-dihydro-7H-pyrido[1,2,3-d,e][1,4]benzoxazin-6-carbonsäure, 10-[(3aRS,4RS)-4-Amino-2,3,3a,4,5,6-hexahydro-1H-isoindol-2-yl]-9-fluor-3-methyl-7-oxo-2,3-dihydro-7H-pyrido[1,2,3-d,e][1,4]benzoxazin-6-carbonsäure, 7-[(3aRS,4RS)-4-Amino-2,3,3a,4,5,6-hexahydro-1H-isoindol-2-yl]-6-fluor-4-oxo-4H-[1,3]thiazeto[3,2-a]chinolin-3-carbonsäure, 7-[(3aRS,4RS)-4-Amino-2,3,3a,4,5,6-hexahydro-1H-isoindol-2-yl]-6-fluor-1-methyl-4-oxo-4H-[1,3]thiazeto[3,2-a]chinolin-3-carbonsäure, 7-[(3aRS,4RS)-4-Amino-2,3,3a,4,5,6-hexahydro-1H-isoindol-2-yl]-6-fluor-1-fluormethyl-4-oxo-4H-[1,3]thiazeto[3,2-a]chinolin-3-carbonsäure, 7-[(3aRS,4RS)-4-Amino-2,3,3a,4,5,6-hexahydro-1H-isoindol-2-yl]-1-cyclopropyl-6-fluor-3-nitroacetyl-4-oxo-1,4-dihydrochinolin, 7-[(3aRS,4RS)-4-Amino-2,3,3a,4,5,6-hexahydro-1H-isoindol-2-yl]-1-cyclopropyl-6-fluor-8-methoxy-3-nitroacetyl-4-oxo-1,4-dihydrochinolin, 7-[(3aRS,4RS)-4-Amino-2,3,3a,4,5,6-hexahydro-1H-isoindol-2-yl]-1-cyclopropyl-6,8-difluor-3-nitroacetyl-4-oxo-1,4-dihydrochinolin, 7-[(3aRS,4RS)-4-Amino-2,3,3a,4,5,6-hexahydro-1H-isoindol-2-yl]-1-cyclopropyl-8-chlor-6-fluor-3-nitroacetyl-4-oxo-1,4-dihydrochinolin, 7-[(3aRS,4RS)-4-Amino-2,3,3a,4,5,6-hexahydro-1H-isoindol-2-yl]-1-cyclopropyl-3-(diethoxycarbonyl)acetyl-6-fluor-4-oxo-1,4-dihydrochinolin, 7-[(3aRS,4RS)-4-Amino-2,3,3a,4,5,6-hexahydro-1H-isoindol-2-yl]-1-cyclopropyl-3-(diethoxycarbonyl)acetyl-6-fluor-8-methoxy-4-oxo-1,4-dihydrochinolin, 7-[(3aRS,4RS)-4-Amino-2,3,3a,4,5,6-hexahydro-1H-isoindol-2-yl]-1-cyclopropyl-3-(diethoxycarbonyl)acetyl-6,8-difluor-4-oxo-1,4-dihydrochinolin, 7-[(3aRS,4RS)-4-Amino-2,3,3a,4,5,6-hexahydro-1H-isoindol-2-yl]-8-chlor-1-cyclopropyl-3-(diethoxycarbonyl)acetyl-6-fluor-4-oxo-1,4-dihydrochinolin, 10-[(3aRS,4RS)-4-Amino-2,3,3a,4,5,6-hexahydro-1H-isoindol-2-yl]-9-fluor-2,3-dihydro-3(S)-methyl-7-oxo-7H-pyrido[1,2,3-d,e][1,4]benzoxazin-6-carbonsäure, 10-[(3aSR,4RS)-4-Amino-2,3,3a,4,5,6-hexahydro-1H-isoindol-2-yl]-9-fluor-2,3-dihydro-3(S)-methyl-7-oxo-7H-pyrido[1,2,3-d,e][1,4]benzoxazin-6-carbonsäure, 7-[(3aRS,4RS)-4-Amino-2,3,3a,4,5,6-hexahydro-1H-isoindol-2-yl]-1-cyclopropyl-8-fluor-1,4-dihydro-4-oxo-3-chinolincarbonsäure, 7-[(3aSR,4RS)-4-Amino-2,3,3a,4,5,6-hexahydro-1H-isoindol-2-yl]-1-cyclopropyl-8-fluor-1,4-dihydro-4-oxo-3-chinolincarbonsäure.

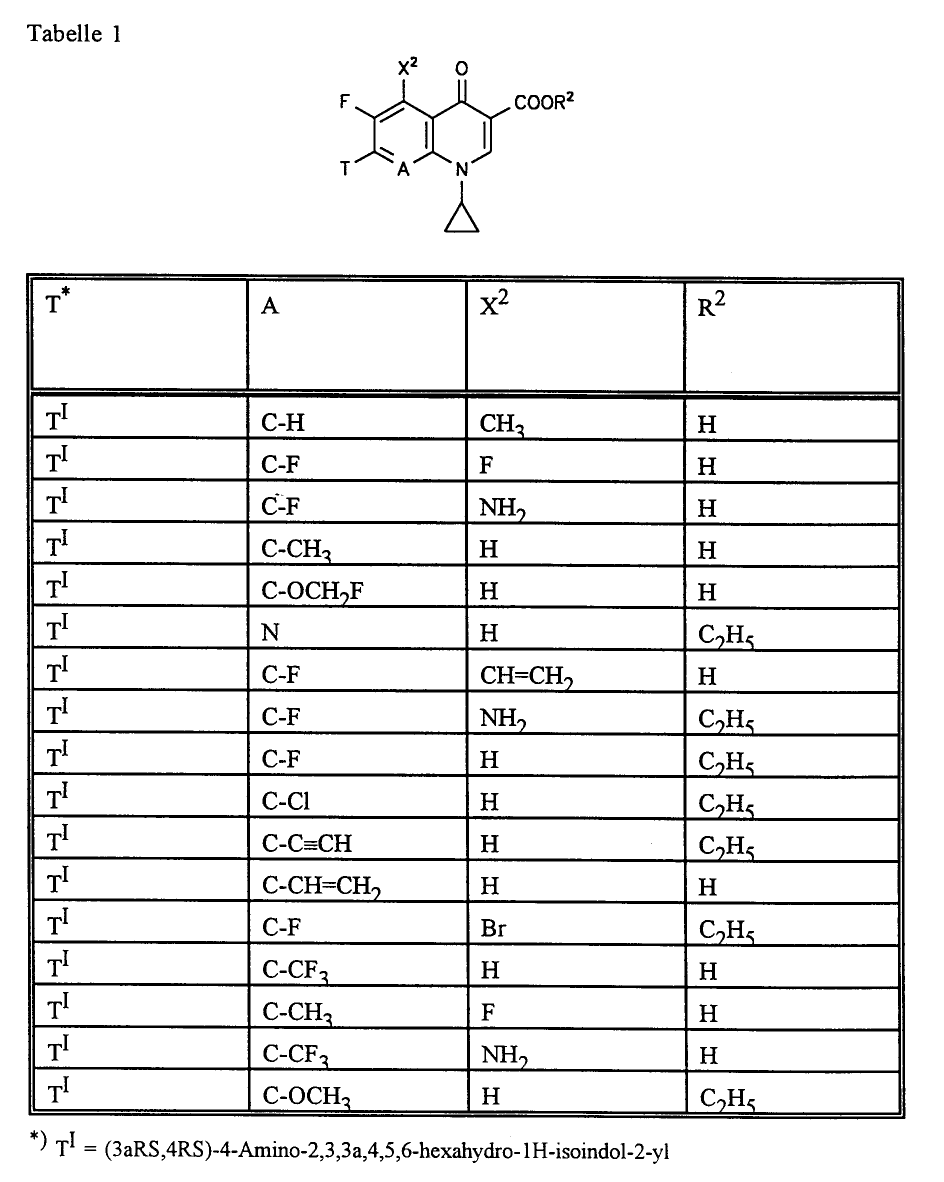

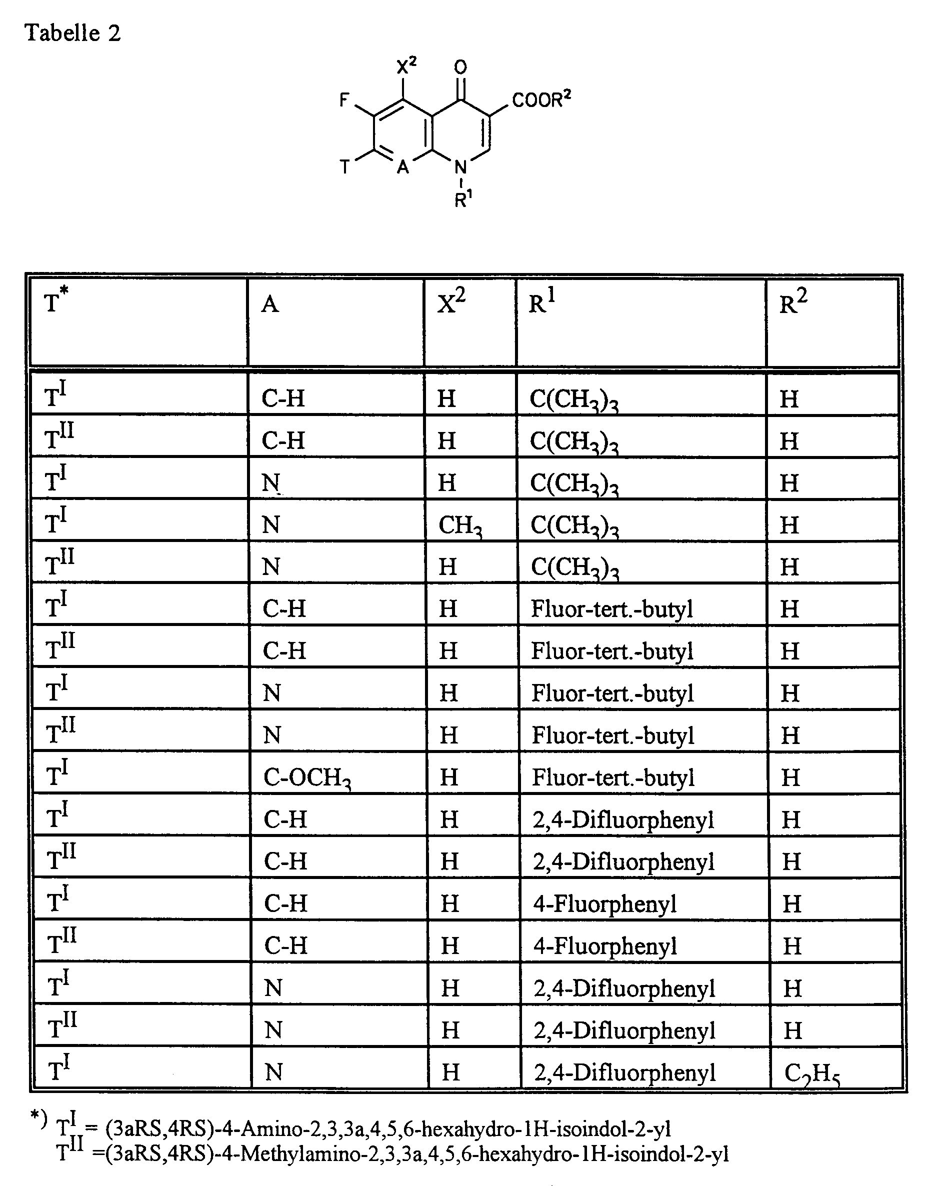

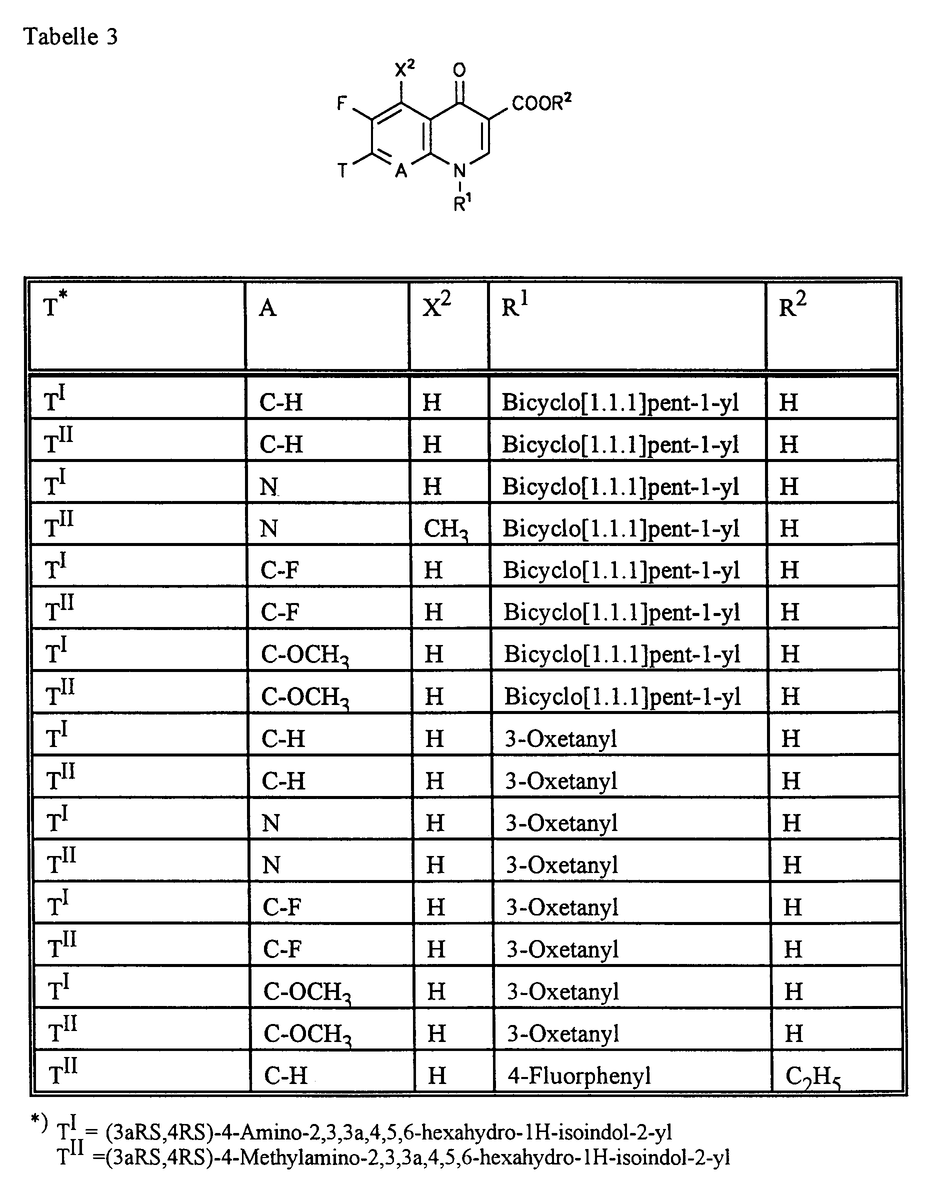

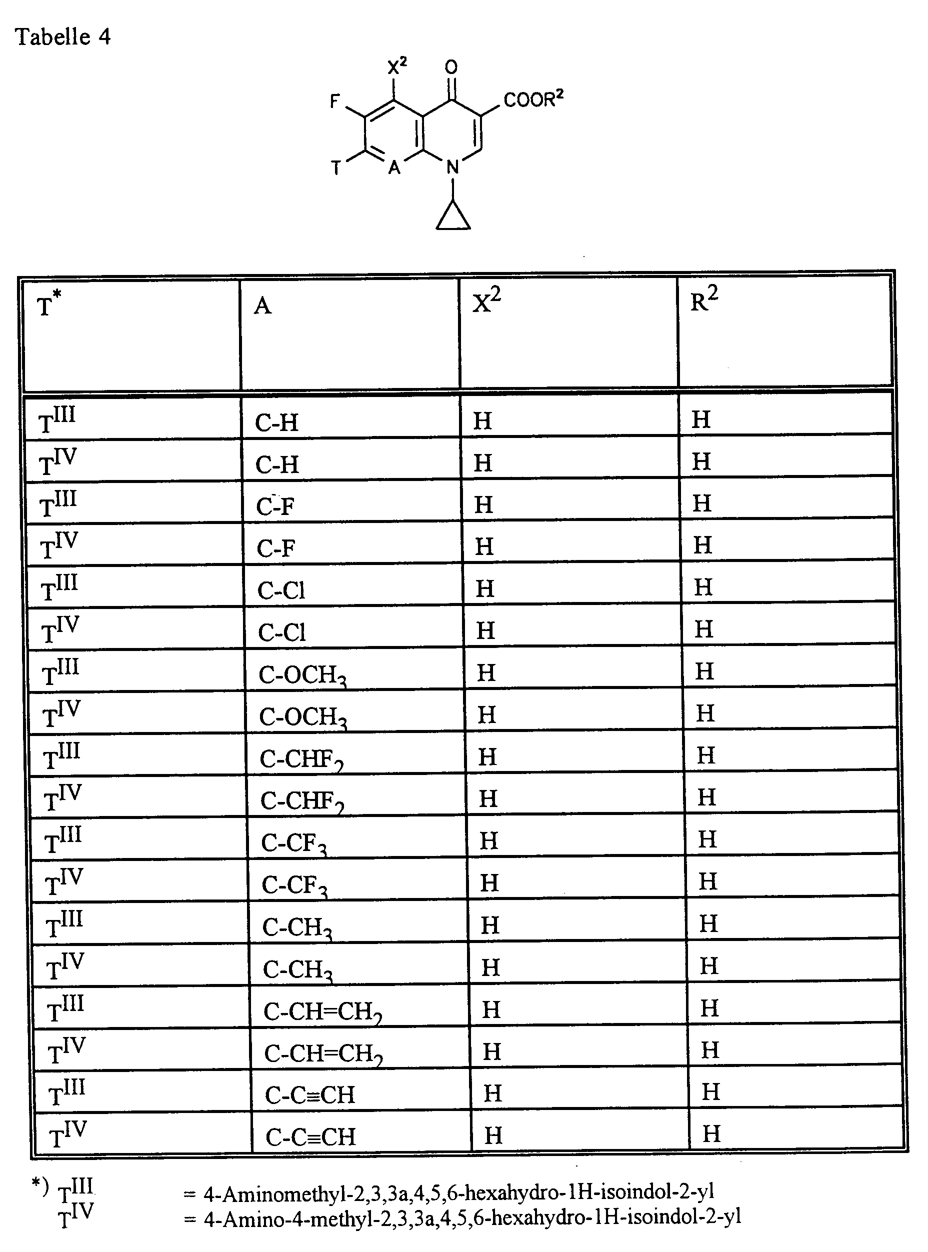

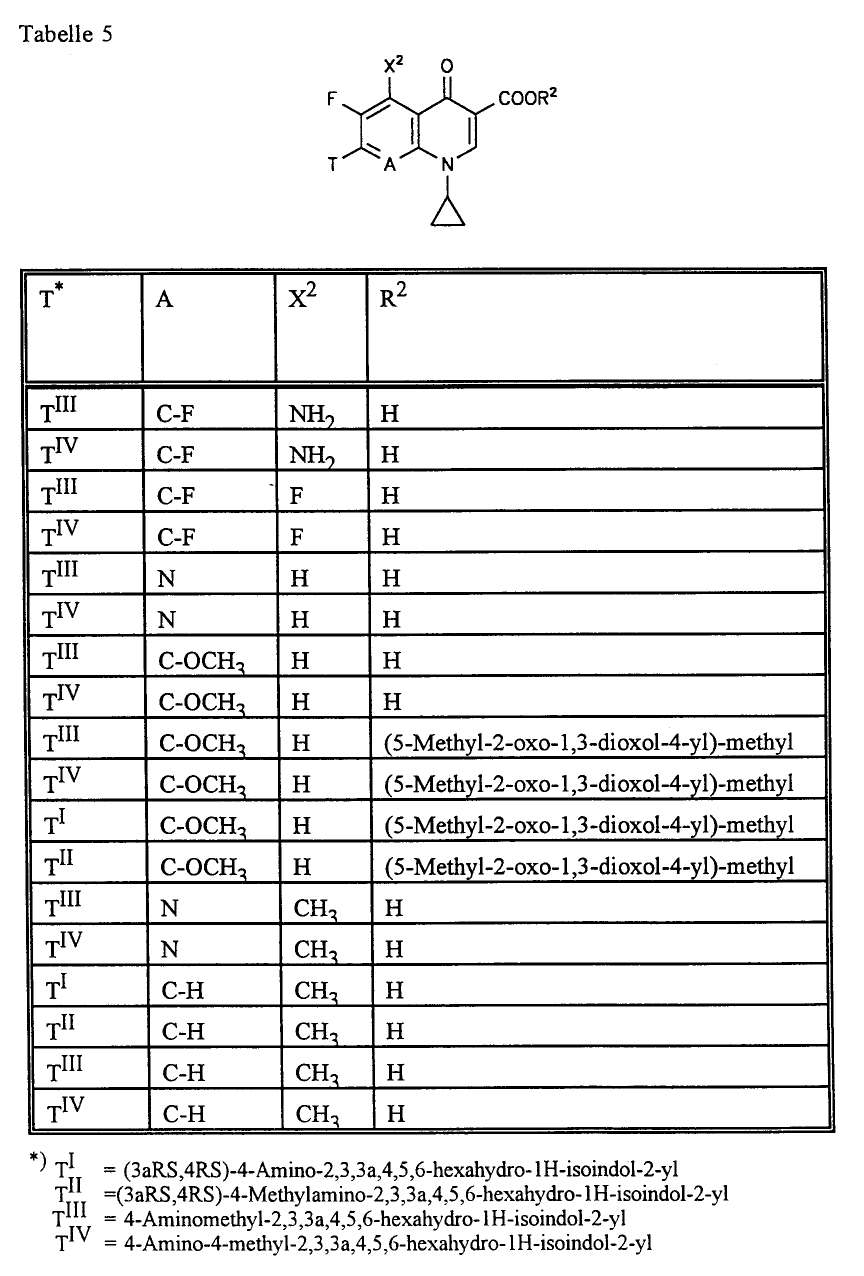

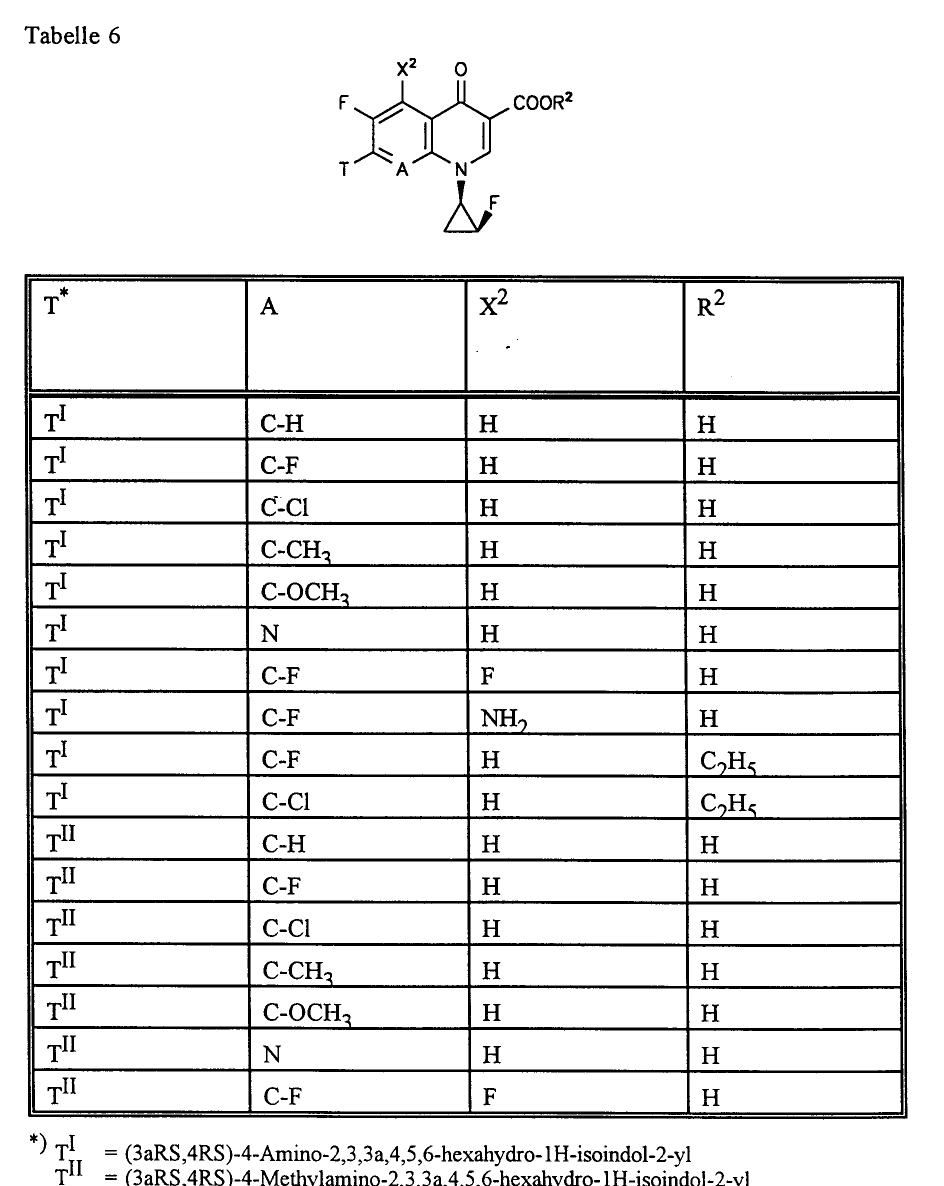

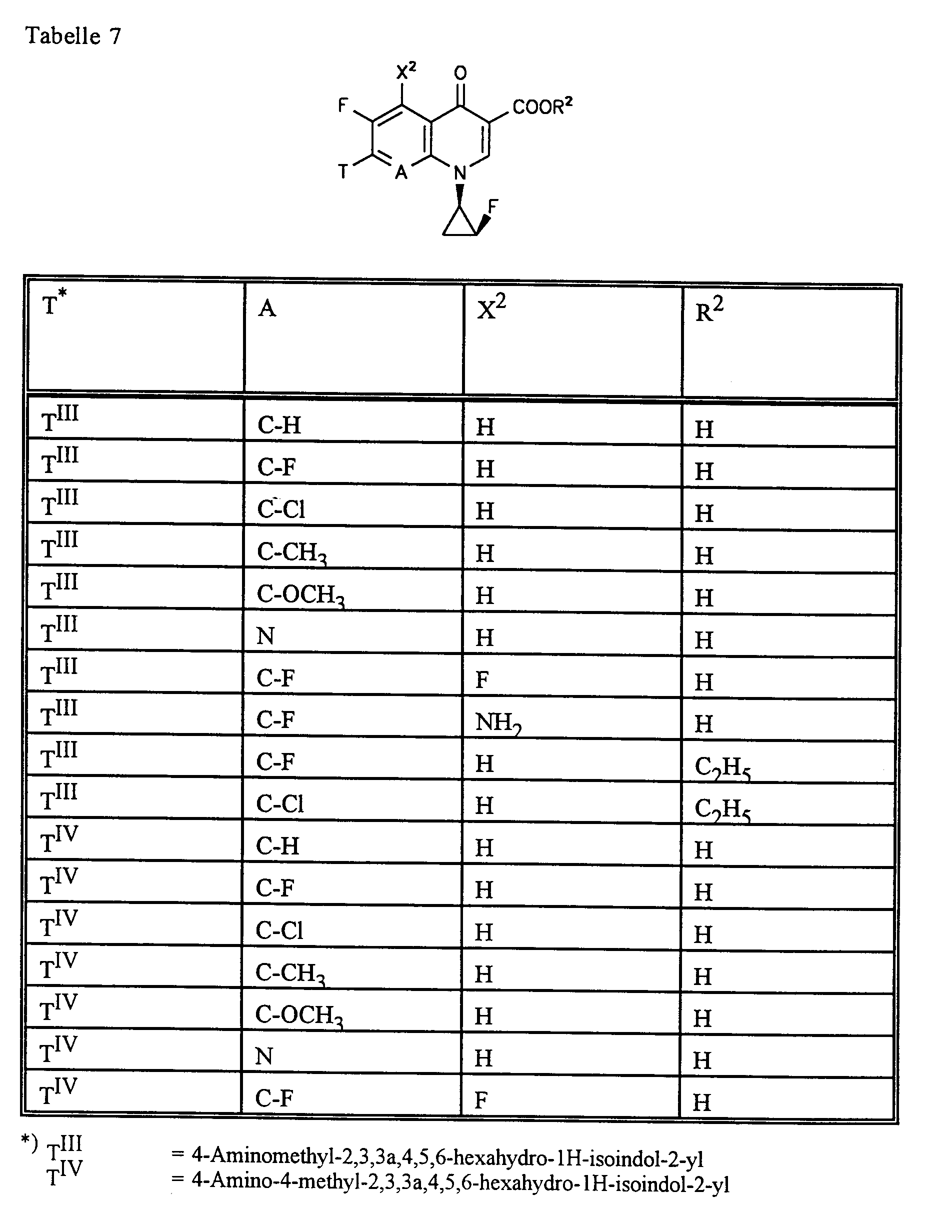

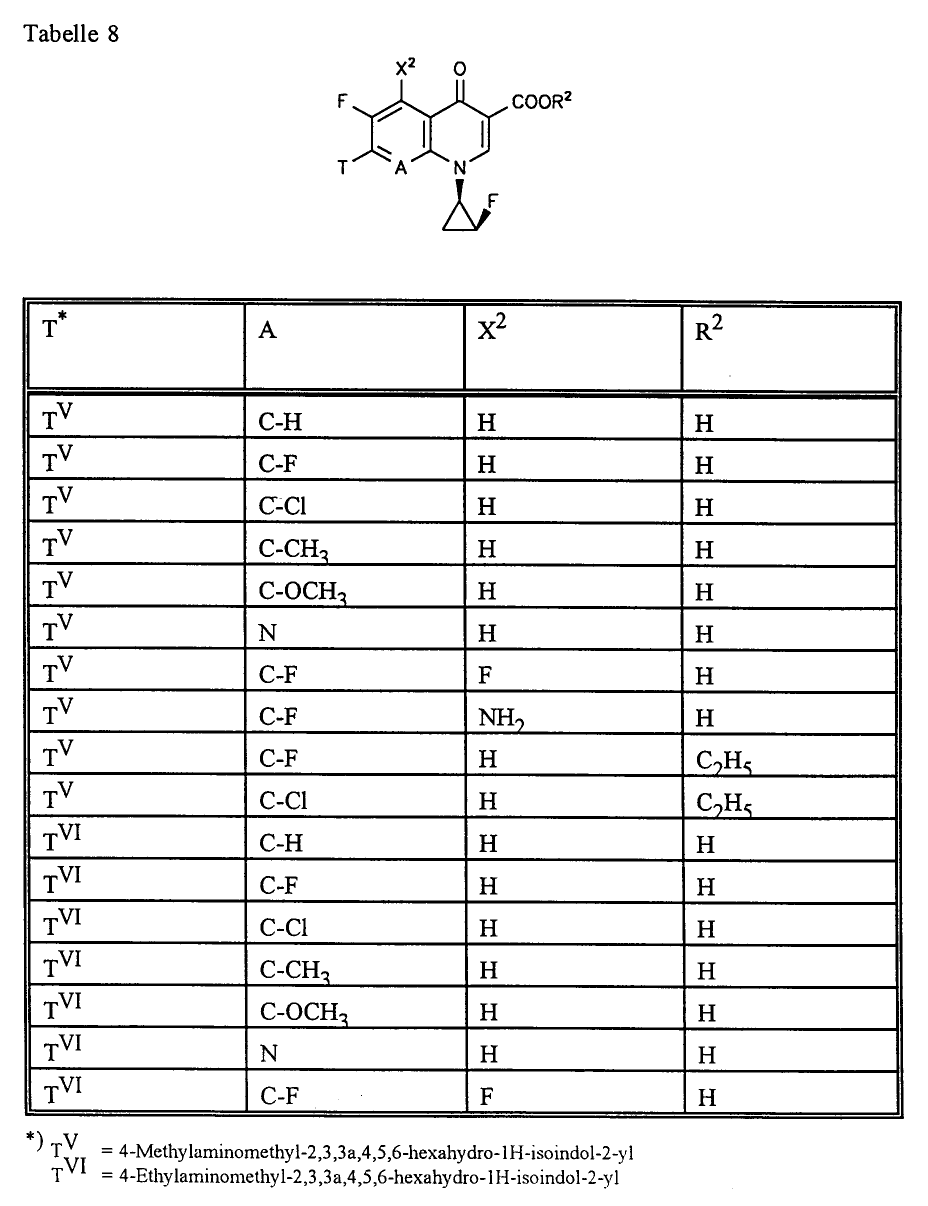

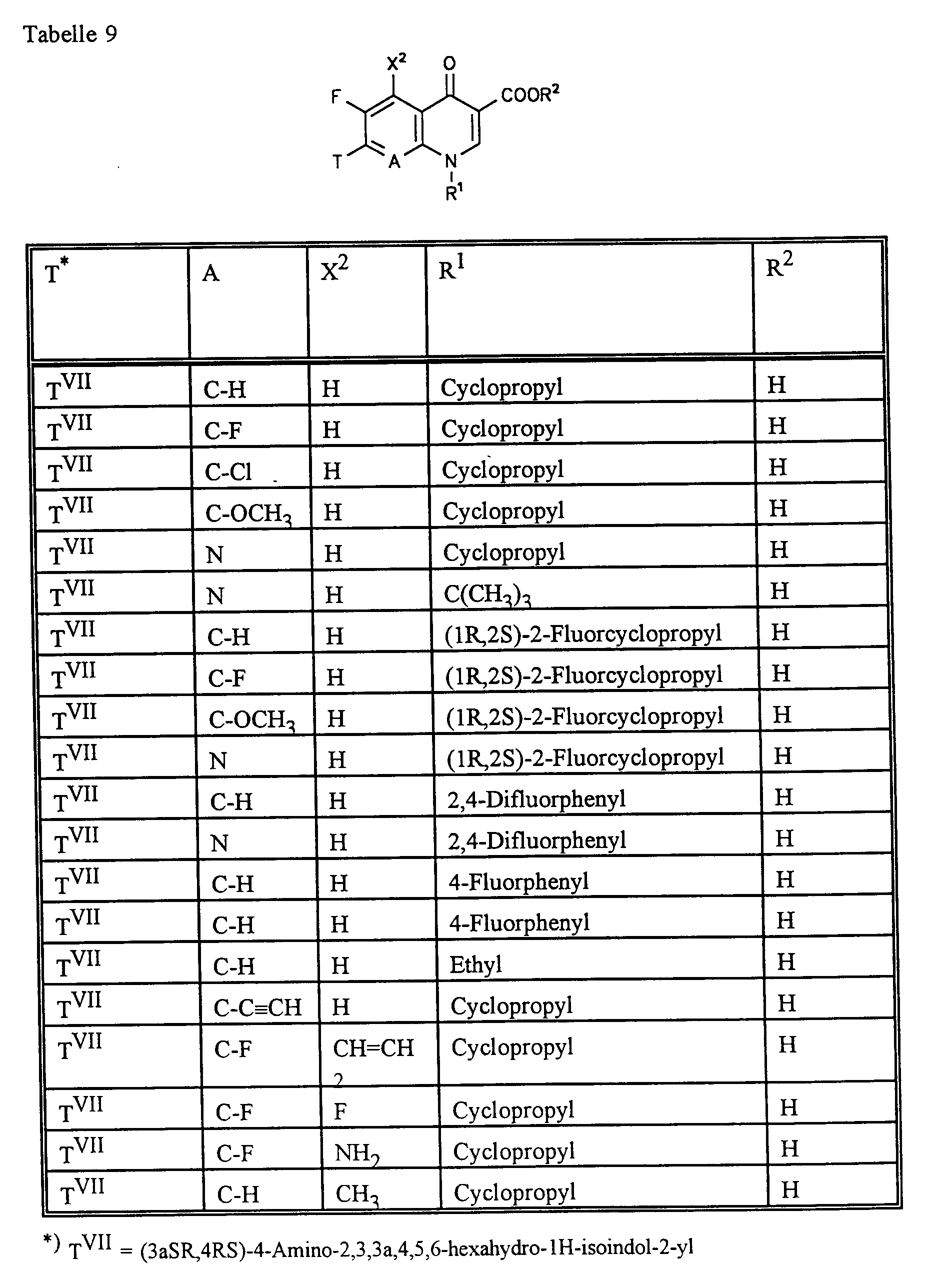

Die erfindungsgemäßen Verbindungen wirken stark antibiotisch und zeigen bei geringer Toxizität ein breites antibakterielles Spektrum gegenüber grampositiven und gramnegativen Keimen, vor allem auch gegenüber solchen, die gegen verschiedene Antibiotika, wie zum Beispiel Penicilline, Cephalosporine, Aminoglykoside, Sulfonamide, Tetracycline sowie gegen handelsübliche Chinolone, resistent sind. Die erfindungsgemäßen Verbindungen zeichnen sich inbesondere dadurch aus, daß sie im Vergleich zu den Verbindungen nach dem Stand der Technik geringere Interaktionen mit Säugetier-DNA aufweisen.

Diese wertvollen Eigenschaften ermöglichen ihre Verwendung als chemotherapeutische Wirkstoffe in der Medizin sowie in der Veterinärmedizin. Ferner können sie als Stoffe zur Konservierung von anorganischen und organischen Materialien, zum Beispiel von Polymeren, Schmiermitteln, Farben, Fasern, Leder, Papier und Holz, von Lebensmitteln und von Wasser verwendet werden.

Die erfindungsgemäßen Verbindungen sind gegen ein sehr breites Spektrum von Mikroorganismen wirksam. Mit ihrer Hilfe können gramnegative und grampostive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch eine verstärkte Wirkung auf ruhende Keime aus. Bei ruhenden Bakterien, also Bakterien, die kein nachweisbares Wachstum zeigen, wirken die Verbindungen stark bakterizid. Dies bezieht sich nicht nur auf die einzusetzende Menge, sondern auch auf die Geschwindigkeit der Abtötung. Solche Ergebnisse konnten bei grampositiven und gramnegativen Bakterien, insbesondere bei Staphylococcus aureus, Pseudomonas aeruginosa, Enterococcus faecalis und Escherichia coli beobachtet werden.

Besonders wirksam sind die erfindungsgemäßen Verbindungen gegen typische und atypische Mykobakterien und Helicobacter pylori sowie gegen bakterienähnliche Mikroorganismen, wie zum Beispiel Mykoplasmen und Rickettsien. Sie sind daher besonders gut in der Human- und Tiermedizin zur Prophylaxe und Chemotherapie von lokalen und systemischen Infektionen geeignet, die durch diese Erreger hervorgerufen werden.

Die Verbindungen eignen sich ferner besonders gut zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die erfindungsgemäßen Verbindungen können auch mit β-Lactamderivaten wie zum Beispiel Cephalosporinen oder Penemen über kovalente Bindungen zu sogenannten Dual-Action-Derivaten verknüpft werden.

In den Tabellen 9 und 10 sind die minimalen Hemmkonzentrationen (MHK-Werte) als Maß für die antibakterielle Wirksamkeit und die ID₅₀-Werte als Maß für die Interaktionen mit Säugetier-DNA einer Substanz sowohl für erfindungsgemäße Verbindungen als auch für Referenzverbindungen aus dem Stand der Technik (EP 520 240) aufgeführt. Diese Daten belegen, daß die erfindungsgemäßen Verbindungen bei hoher antibakterieller Wirksamkeit geringere Interaktionen mit Säugetier-DNA aufweisen.

Die minimalen Hemmkonzentrationen (MHK) wurden durch ein Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Mutipoint-Inokulator (Denley) beimpft. Zum Beimpfen wurden Übernachtkulturen der Erreger verwandt, die zuvor so verdünnt wurden, daß jeder Impfpunkt circa 10⁴ koloniebildende Partikel enthielt. Die beimpften Agarplatten wurden bei 37°C bebrütet und das Keimwachstum nach circa 20 Stunden abgelesen. Der MHK-Wert (µg/ml) gibt die niedrigste Wirkstoffkonzentration an, bei der mit bloßem Auge kein Wachtum zu erkennen war.

Unter ID₅₀ versteht man die Konzentration eines Stoffes, bei der die DNA-Synthese in Zellen aus Ovarien des chinesischen Hamsters (CHO-KI) um 50 % gehemmt wird. Dieser Wert wird nach Inkubation der entsprechenden Substanzen in abfallenden Verdünnungsstufen über definierte Zeiträume bestimmt. Dazu wird mittels fluorophotometrischer Methoden die DNA-Synthese in CHO-KI-Zellen im Vergleich zu Kontrollen ermittelt.

**Tabelle 9**

| MHK-Werte (µg/ml) und ID₅₀-Werte von erfindungsgemäßen Wirkstoffen | | | | | |
|---|---|---|---|---|---|
| Species | Strain | Beipiel | | | |
| | | 1 | 2 | 3 | 4 |
| E. coli | Neumann | ≤0,015 | ≤0,015 | ≤0,015 | ≤0,015 |
| Staph. aureus | 133 | ≤0,015 | ≤0,015 | ≤0,015 | ≤0,015 |
| Staph. aureus | ICB 25701 | ≤0,015 | 0,125 | ≤0,015 | 0,03 |
| Ps. aeruginosa | Walter | 0,125 | 1 | 0,5 | 0,25 |
| ID₅₀ (µg/ml) | | 4 | 2 | 0,1 | 2 |

**Tabelle 10**

| MHK-Werte (µg/ml) und ID₅₀-Werte von Wirkstoffen aus dem Stand der Technik | | | | |
|---|---|---|---|---|
| Species | Strain | Beipiele aus EP 520 240 *⁾ | | |
| | | 17 B | 19 | 18 A |
| E.coli | Neumann | ≤0,015 | ≤0,015 | ≤0,015 |
| Staph. aureus | 133 | ≤0,015 | ≤0,015 | ≤0,015 |
| Staph. aureus | ICB 25701 | 0,03 | 1 | ≤0,015 |
| Ps. aeruginosa | Walter | 0,25 | 1 | 0,25 |
| ID₅₀ (µg/ml) | | 0,01 | 1 | 0,06 |

| | | | | |
|---|---|---|---|---|
| *⁾ Referenzverbindungen aus EP 520 240: 17 B: 7-(4-Amino-1,3,3a,4,7,7a-hexahydro-isoindol-2-yl)-1-cyclopopyl-6,8-difluor-1,4-dihydro-4-oxo-3-chinolincarbonsäure, 19: 7-(4-Amino-1,3,3a-4,7,7a-hexahydro-isoindol-2-yl)-1-cyclopopyl-6-fluor-1,4-dihydro-4-oxo-3-chinolincarbonsäure, 18 A : 7-(4-Amino-1,3,3a,4,7,7a-hexahydro-isoindol-2-yl)-8-chlor-1-cyclopopyl-6-fluor-1,4-dihydro-4-oxo-3-chinolincarbonsäure. | | | | |

### Herstellung der Zwischenprodukte

### Beispiel Z 1

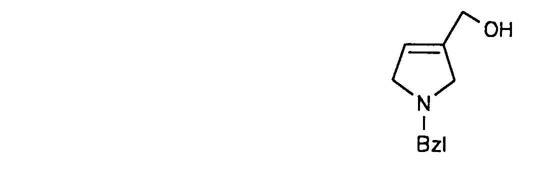

Es werden 87,0 g (0,38 mol) 1-Benzyl-2,5-dihydro-1H-pyrrol-3-carbonsäureethylester in 430 ml Tetrahydrofuran vorgelegt, auf -70°C abgekühlt und tropfenweise mit 1200 ml 1 molarer Lösung von Diisobutylaluminiumhydrid in Hexan versetzt. Man rührt 3 Stunden bei -70°C und weitere 15 Stunden bei Raumtemperatur nach. Die Mischung wird auf 0°C gekühlt und durch Zugabe von 390 ml gesättigter Ammoniumchloridlösung und 390 ml 6 normaler Schwefelsäure hydrolisiert. Die wäßrige Phase wird abgetrennt, mit Kaliumhydroxid basisch gestellt und mehrfach mit tert.-Butylmethylether extrahiert. Die vereinigten organischen Extrakte werden getrocknet, eingeengt und destilliert.
Ausbeute: 58,0 g (81% der Theorie) 1-Benzyl-2,5-dihydro-3-hydroxymethyl-1H-pyrrol,
Siedepunkt: 120-122°C/0,1 mbar,
¹H-NMR (CDCl₃): δ 7,50-7,18 (m, 5H), 5,55 (m, 1H), 4,10 (m, 2H), 3,80 (s, 2H), 3,70-3,35ppm (m, 5H).

### Beispiel Z 2

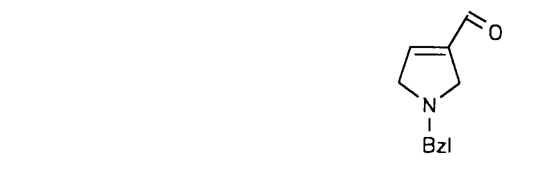

Es werden 64,3 g (0,49 mol) Oxalylchlorid in 580 ml Dichlormethan gelöst und auf -70°C abgekühlt. Hierzu tropft man zuerst eine Lösung aus 76,5 g Dimethylsulfoxid in 174 ml Dichlormethan, dann eine Lösung aus 58,0 g (0,31 mol) 1-Benzyl-2,5-dihydro-3-hydroxymethyl-1H-pyrrol in 174 ml Dichlormethan und anschließend 198 g Triethylamin. Die Mischung wird 30 Minuten nachgerührt, auf Raumtemperatur erwärmt und auf 600 g Eis gegossen. Die organische Phase wird abgetrennt, die wäßrige Phase mit Kaliumcarbonat stärker basisch gestellt und mehrfach mit Dichlormethan extrahiert. Die vereinigten organischen Phasen werden getrocknet, eingeengt und ohne weitere Reinigung für die nächste Reaktion verwendet.
Ausbeute (roh): 59,5 g 1-Benzyl-2,5-dihydro-1H-pyrrol-3-carbaldehyd,
¹H-NMR (CDCl₃): δ 9,74 (s, 1H), 7,60-7,10 (m, 5H), 6,85 (m, 1H), 3,81 (s, 2H), 3,80-3,60 ppm (m, 4H).

### Beispiel Z 3

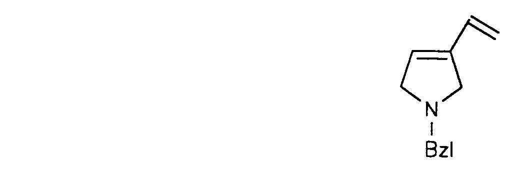

Es werden 129 g (0,36 mol) Methyltriphenylphosphoniumbromid in 828 ml Tetrahydrofuran vorgelegt, auf -10°C abgekühlt, 111 g einer 23%igen n-Butyllithiumlösung in Hexan zugetropft, 20 Minuten bei - 10°C und 90 Minuten bei -70°C nachgerüht. Anschließend wird eine Lösung von 59,1 g (0,31 mol) 1-Benzyl-2,5-dihydro-1H-pyrrol-3-carbaldehyd in 197 ml Tetrahydrofuran zugetropft und über Nacht auf Raumtemperatur erwärmt. Die Mischung wird in 2000 ml einer gesättigten Natriumchloridlösung gegossen und mehrfach mit Petrolether extrahiert. Die vereinigten organischen Phasen werden getrocknet, ausfallendes Triphenylphosphinoxid abgesaugt, erneut eingeengt und ohne weitere Reinigung für die nächste Reaktion verwendet.
Ausbeute: 57,0 g (82% der Theorie, Reinheit 83% nach GC) 1-Benzyl-2,5-dihydro-3-vinyl-1H-pyrrol,
¹H-NMR (CDCl₃): δ 7,40-7,20 (m, 5H), 6,48 (dd, 1H), 5,73 (s, 1H), 5,03 (dd, 2H), 3,85 (s, 2H), 3,58ppm (s, 4H).

### Beispiel Z 4

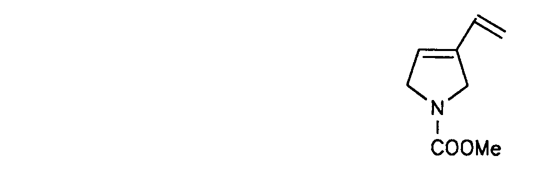

Es werden 20,0 g (0.08 mol, 73%ig) 1-Benzyl-2,5-dihydro-3-vinyl-1H-pyrrol gelöst in 180 ml Dichlormethan mit 13,8 g Natriumcarbonat vermischt und auf 0°C abgekühlt. Man tropft eine Lösung von 12,3 g Chlorameisensäuremethylester in 40 ml Dichlormethan zu und rührt 15 Stunden bei Raumtemperatur nach. Der Feststoff wird abfiltriert, mehrfach mit Dichlormethan ausgewaschen und die Filtrate vereinigt. Sie werden mit Wasser gewaschen, getrocknet, eingeengt und an Kieselgel mit einer Mischung von Essigsäureethylester und Petrolether (1:10) chromatographiert.
Ausbeute: 7,70 g (46% der Theorie) 2,5-Dihydro-1-methoxycarbonyl-3-vinyl-1H-pyrrol,
¹H-NMR (CDCl₃): δ 6,49 (dd, 1H), 5,80-5,67 (m, 1H), 5,25-5,00 (m, 2H), 4,35-4,15 (m, 4H), 3,74ppm (d, 3H).

### Beispiel Z 5

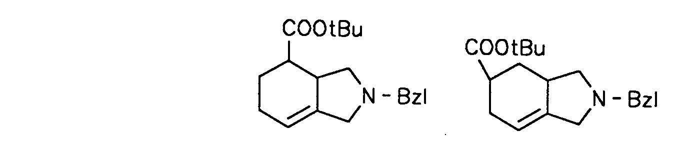

Es werden 20,0 g (0,08 mol) 1-Benzyl-2,5-dihydro-3-vinyl-1H-pyrrol in 200 ml Xylol gelöst und mit einer Lösung aus 39,4 g (0,31 mol) Acrylsäure-tert.-butylester in 200 ml Xylol versetzt. Die Mischung wird über Nacht zum Rückfluß erhitzt, abgekühlt und eingeengt.
Ausbeute: 17,6 g (0,06 mol) einer Mischung von vier Isomeren, die sich chromatographisch auftrennen läßt. Säulenchromatographie an Kieselgel mit Gemischen von Essigsäureethylester und Petrolether (1:30 bis 1:5) ermöglicht es, den (5RS,6RS)-8-Benzyl-8-azabicyclo[4.3.0]non-1-en-5-carbonsäure-tert.-butylester [(3aRS,4RS)-2-Benzyl-2,3,3a,4,5,6-hexahydro-1H-isoindol-4-carbonsäure-tert.-butylester] und den (5RS,6SR)-8-Benzyl-8-azabicyclo[4.3.0]non-1-en-5-carbonsäure-tert.-butylester [(3aRS,4SR)-2-Benzyl-2,3,3a,4,5,6-hexahydro-1H-isoindol-4-carbonsäure-tert.-butylester] von den beiden gebildeten 8-Benzyl-8-azabicyclo[4.3.0]non-1-en-4-carbonsäure-tert.-butylestern abzutrennen.
¹H-NMR des (5RS,6RS)-Isomeren (CDCl₃): δ 7,40-7,20 (m, 5H), 5,40 (m, 1H), 3,66 (q, 2H), 3,56 (dm, 1H), 3.29 (dd, 1H), 2,92 (dm, 1H), 2,70-2,80 (m, 1H), 2,20-1,90 (m, 5H), 1,75-1,45 (m, 1H), 1,43 ppm (s, 9H).
¹H-NMR des (5RS,6SR)-Isomeren (CDCl₃): δ 7,42-7,20 (m, 5H), 5,43 (m, 1H), 3,67 (q, 2H), 3,56 (dm, 1H), 3.06-2.70 (m, 3H), 2,42-1,95 (m, 4H), 1,85-1,65 (m, 2H), 1,43ppm (s, 9H).

### Beispiel Z 6

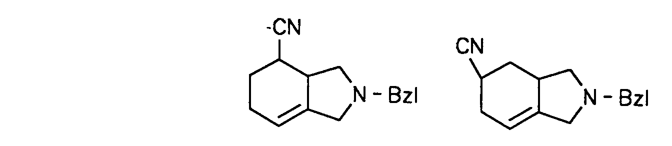

Es werden 7,00 g (0,04 mol) 1-Benzyl-2,5-dihydro-3-vinyl-1H-pyrrol in 50 ml Xylol gelöst und mit einer Lösung aus 5,00 g (0.09 mol) Acrylnitril in 50 ml Xylol versetzt. Die Mischung wird über Nacht zum Rückfluß erhitzt, abgekühlt und eingeengt.
Ausbeute: 3,7 g (41% der Theorie) einer Mischung von vier Isomeren, die sich chromatographisch auftrennen läßt. Säulenchromatograghie an Kieselgel mit Gemischen von Essigsäureethylester und Petrolether (1:30 bis 1:5) ermöglicht es, das 8-Benzyl-8-azabicyclo[4.3.0]non-1-en-5-carbonsaurenitril vom 8-Benzyl-8-azabicyclo[4.3.0]non-1-en-4-carbonsäurenitril abzutrennen.
¹H-NMR von (5RS,6RS)-8-Benzyl-8-azabicyclo[4.3.0]non-1-en-5-carbonsäurenitril (CDCl₃): δ 7,40-7,20 (m, 5H), 5,45 (m, 1H), 3,78 (d, 1H), 3,58 (d, 1H), 3,55 (dm, 1H), 3,22 (dd, 1H), 2,90 (dm, 1H), 2,80-2,65 (m, 4H), 1,90-1,65 ppm (m, 1H).

### Beispiel Z 7

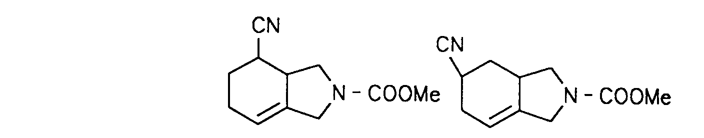

Es werden 3,00 g (0,01 mol) einer Mischung von 8-Benzyl-8-azabicyclo[4.3.0]non-1-en-5-carbonsäurenitril und 8-Benzyl-8-azabicyclo[4.3.0]non-1-en-4-carbonsäurenitril in 20 ml Dichlormethan gelöst, mit 1,93 g Natriumcarbonat verrührt und mit 2,00 g Chlorameisensäureethylester, gelöst in 3 ml Dichlormethan, versetzt. Man rührt 15 Stunden bei Raumtemperatur nach, filtriert den Feststoff ab und wäscht mehrfach mit Dichlormethan nach. Die vereinigten Filtrate werden mit Wasser gewaschen, getrocknet, eingeengt und mit einer Mischung aus Essigsäureethylester und Petrolether (1:4) an Kieselgel chromatographiert.
Ausbeute: 1,1 g (40 % der Theorie) einer Mischung aus 8-Methoxycarbonyl-8-azabicyclo[4.3.0]non-1-en-4-carbonsäurenitril und 8-Methoxycarbonyl-8-azabicyclo[4.3.0]non-1-en-5-carbonsäurenitril.

### Beispiel Z 8

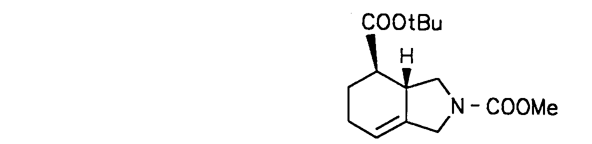

Es werden 23,0 g (0,07 mol) (5RS,6RS)-8-Benzyl-8-azabicyclo[4.3.0]non-1-en-5-carbonsäure-tert.-butylester in 180 ml Dichlormethan mit 12,5 g (0,12 mol) Natriumcarbonat vermischt, auf 0°C abgekühlt und tropfenweise mit einer Lösung von 11,9 g (0,12 g) Chlorameisensäuremethylester in 45 ml Dichlormethan versetzt. Nach 30 Minuten bei 0°C erwärmt man auf Raumtemperatur und rührt über Nacht nach. Der Feststoff wird filtriert und ausgewaschen, die vereinigten organischen Filtrate mit Wasser gewaschen, getrocknet, eingeengt und das während der Reaktion gebildete Benzylchlorid destillativ entfernt.
Ausbeute: 17,0 g (76 % der Theorie) (5RS,6RS)-8-Methoxycarbonyl-8-azabicyclo[4.3.0]non-1-en-5-carbonsäure-tert.-butylester,
¹H-NMR (CDCl₃): δ 5,60 (m, 1H), 4,05-3,75 (m, 3H), 3,70 (s, 3H), 2,90-2,72 (m, 2H), 2,60-2,42 (m, 2H), 2,35-2,10 (m, 3H), 1,45 ppm (s, 9H).

### Beispiel Z 9

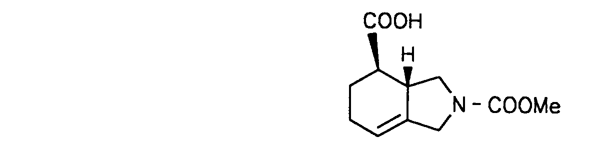

Es werden 17,0 g (0,06 mol) (5RS,6RS)-8-Methoxycarbonyl-8-azabicyclo[4.3.0]non-1-en-5-carbonsäure-tert.-butylester in 420 ml Dichlormethan bei 0°C vorgelegt, tropfenwise mit 120 ml Trifluoressigsäure versetzt und drei Stunden bei Raumtemperatur nachgerührt. Die Mischung wird eingeengt, erneut in Dichlormethan aufgenommen und mehrfach mit Wasser gewaschen. Der Rückstand wird an Kieselgel mit einer Mischung aus Cyclohexan und Aceton (3:1) filtriert.
Ausbeute: 7,9 g (58 % der Theorie) (5RS,6RS)-8-Methoxycarbonyl-8-azabicyclo[4.3.0]non-1-en-5-carbonsäure,
Schmelzpunkt: 144-147°C,
¹H-NMR (CDCl₃): δ 10,5-9,20 (m, 1H), 5,65 (dm, 1H), 4,10-3,75 (m, 3H), 3,71 (s, 3H), 3,00-2,90 (m, 1H), 2,90-2,80 (m, 1H), 2,30-2,14 (m, 4H), 1,75-1,65 ppm (m, 1H).

### Beispiel Z 10

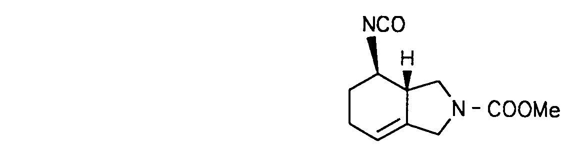

Es werden 8,50 g (0.04 mol) (5RS,6RS)-8-Methoxycarbonyl-8-azabicyclo[4.3.0)non-1-en-5-carbonsäure in 200 ml Toluol vorgelegt und mit 4,20 g Triethylamin und 12,5 g Diphenylphosphorylazid vermischt. Man erwärmt 5 Stunden zum Rückfluß, gießt die abgekühlte Lösung auf Eiswasser, trennt die organische Phase ab und extrahiert noch mehrfach mit Toluol. Die vereinigten Extrakte werden getrocknet, eingeengt und ohne weitere Reinigung für die folgende Umsetzung verwendet.
Rohausbeute: 10,6 g eines braunen Öls, (5RS,6RS)-8-Methoxycarbonyl-8-azabicyclo[4.3.0]non-1-en-5-ylisocyanat,
IR: 2250 cm⁻¹,
¹H-NMR (CD₃OD): δ 5,62 (m, 1H), 4,08-3,76 (m, 3H), 3,67 (s, 3H), 3,42 (ddd, 1H), 2,96 (tm, 1H), 2,80-2,50 (m, 1H), 2.35-2.15 (m, 2H), 2,02-1,83 (m, 1H), 1,65-1,40 ppm (m, 1H).

### Beispiel Z 11

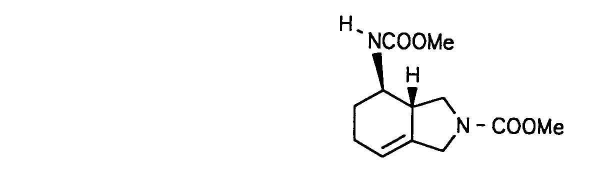

Es werden 10,6 g rohes (5RS,6RS)-8-Methoxycarbonyl-8-azabicyclo[4.3.0]non-1-en-5-ylisocyanat in 200 ml Methanol gelöst und 15 Stunden zum Rückfluß erhitzt. Die erkaltete Mischung wird eingeengt und an Kieselgel mit einer Mischung aus Cyclohexan und Aceton (3:1) chromatographiert.
Ausbeute: 6,11 g (64 % der Theorie, bezogen auf die Säure) (5RS,6RS)-5 Methoxycarbonylamino-8-azabicyclo[4.3.0]non-1-en-8-carbonsäuremethylester,
Schmelzpunkt: 118-121°C,
¹H-NMR (CDCl₃): δ 5,59 (d, 1H), 4,65 (m, 1H), 4,07-3,85 (m, 3H), 3,72 (s, 3H), 3,68 (s, 3H), 3,50 (m, 1H), 3,08 (qm, 1H), 2,52 (m, 1H), 2,35-2,15 (m, 2H), 2,05-1,95 (m, 1H), 1,53-1,41 ppm (m, 1H).

### Beispiel Z 12

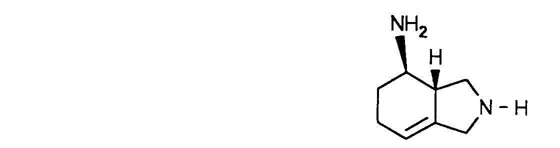

Es werden 6,00 g (0,02 mol) (5RS,6RS)-5-Methoxycarbonylamino-8-azabicyclo[4.3.0]non-1-en-8-carbonsäuremethylester in 20 ml Ethanol gelöst und mit 400 g einer gesättigten Bariumhydroxidlösung versetzt. Die Mischung wird zwei Tage zum Rückfluß erhitzt, abgekühlt, das ausgefallene Bariumcarbonat abfiltriert und das Filtrat mehrfach mit Chloroform extrahiert. Die organischen Phasen werden getrocknet und eingeengt.
Ausbeute: 3,20 g (98 % der Theorie) (5RS,6RS)-5-Amino-8-azabicyclo[4.3.0]non-1-en [(3aRS,4RS)-2,3,3a,4,5,6-Hexahydro-1H-isoindol-4-ylamin],
¹H-NMR (CDCl₃): δ 5,46 (m, 1H), 3,72-3,55 (m, 1H), 3,55-3,33 (m, 2H), 2,65-2,36 (m, 2H), 2,33-2,05 (m, 3H), 1,91-1,76 (m, 1H), 1,64 (s, 3H), 1,53-1,30 ppm (m, 1H).

### Beispiel Z 13

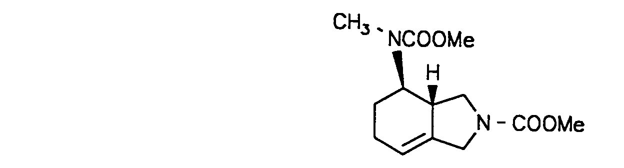

Es werden 240 mg (7,87 mmol) 80%iges Natriumhydrid in 10 ml Tetrahydrofuran vorgelegt, 1,00 g (5RS,6RS)-5-Methoxycarbonylamino-8-azabicyclo[4.3.0]non-1-en-8-carbonsäuremethylester, gelöst in 10 ml Tetrahydrofuran zugetropft und 30 Minuten zum Rückfluß erhitzt. Man läßt auf Raumtemperatur abkühlen, tropft 1,12 g (7,87 mmol) Methyliodid zu und erwärmt 15 Stunden zum Rückfluß. Anschließend kühlt man auf Raumtemperatur, zersetzt überschüssiges Natriumhydrid mit wenig Wasser, gießt auf Eiswasser und extrahiert mehrfach mit Essigester.
Ausbeute: 1,00 g (95% der Theorie).
(5RS,6RS)-Isomer: ¹H-NMR (CDCl₃): δ 5,58 (d, 1H), 4,08-3,89 (m, 3H), 3,85-3,75 (m, 1H), 3,72 (s, 3H), 3,70 (s, 3H), 3,02 (t, 1H), 2,90-2,82 (m, 1H), 2,84 (s, 3H), 2,35-2,25 (m, 2H), 1,80-1,72 ppm (m, 2H).

### Beispiel Z 14

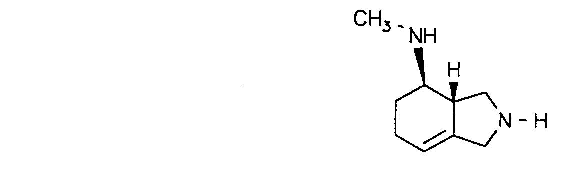

Es werden 1,5 g (6 mmol) (5RS,6RS)-5-(N-Methoxycarbonyl-N-methyl-amino)-8-azabicyclo[4.5.0]non-1-en-8-carbonsäuremethylester in 10 ml Ethanol gelöst und mit 130 g einer gesättigten Bariumhydroxidlösung versetzt. Die Mischung wird zwei Tage zum Rückfluß erhitzt, abgekühlt, das ausgefallene Bariumcarbonat abfiltriert und das Filtrat mehrfach mit Chloroform extrahiert. Die vereinigten organischen Phasen werden getrocknet und eingeengt. Man erhält 0.6 g eines braunen Öls, das an Kieselgel mit einer Mischung aus Dichlormethan/Methanol/wäßriges Ammoniak (14:5:1) chromatographiert wird.
Ausbeute: 0,1 g (9 % der Theorie) (5RS,6RS)-5-Methylamino-8-azabicyclo[4.3.0]non-1-en [(3aRS,4RS)-4-Methylamino-2,3,3a,4,5,6-hexahydro-1H-isoindol],
¹H-NMR (CDCl₃/CD₃OD): δ 5,88 (m, 1H), 4,02-3,85 (m, 3H), 3,18-3,04 (m, 3H), 3,75 (s, 3H), 2,48-2,23 (m, 3H), 1,82-1,72 ppm (m, 1H).

### Beispiel Z 15

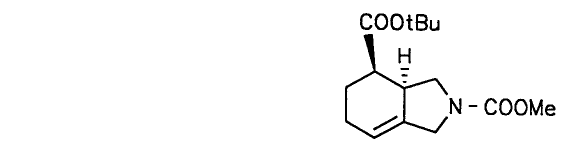

Es werden 17,0 g (0,05 mol) (5RS,6SR)-8-Benzyl-8-azabicyclo[4.3.0]non-1-en-5-carbonsäure-tert.-butylester in 140 ml Dichlormethan gelöst mit 9,4 g (0,09 mol) Natriumcarbonat vermischt, auf 0°C abgekühlt und tropfenweise mit einer Lösung von 8,9 g (0,09 g) Chlorameisensäuremethylester in 35 ml Dichlormethan versetzt. Nach 30 Minuten bei 0°C erwärmt man auf Raumtemperatur und rührt über Nacht nach. Der Feststoff wird filtriert und ausgewaschen, die vereinigten organischen Filtrate mit Wasser gewaschen, getrocknet, eingeengt und das während der Reaktion gebildete Benzylchlorid destillativ entfernt.
Ausbeute: 4,9 g (32% der Theorie) (5RS,6SR)-8-Methoxycarbonyl-8-azabicyclo[4.3.0]non-1-en-5-carbonsäure-tert.-butylester,
¹H-NMR (CDCl₃): δ 5,63 (m, 1H), 4,05-3,90 (m, 2H), 3,82-3,65 (m, 1H), 3,71 (s, 3H), 3,29 (t, 1H), 2,95-2,75 (m, 2H), 2,39-1,92 (m, 3H), 1,89-1,69 (m, 1H), 1,45 ppm (s, 9H).

### Beispiel Z 16

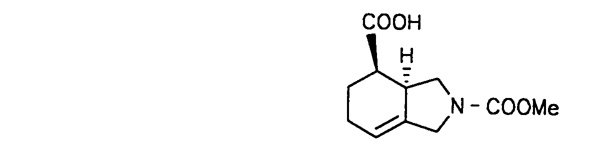

Es werden 4,0 g (0,01 mol) (5RS,6SR)-2-Methoxycarbonyl-2-azabicyclo[4.3.0]non-4-en-8-carbonsäure-tert.-butylester in 100 ml Dichlormethan bei 0°C vorgelegt, tropfenweise mit 30 ml Trifluoressigsäure versetzt und 3 Stunden bei Raumtemperatur nachgerührt. Die Mischung wird eingeengt, erneut in Dichlormethan aufgenommen und mehrfach mit Wasser gewaschen. Der Rückstand wird an Kieselgel mit einer Mischung aus Cyclohexan und Aceton (3:1) filtriert.
Ausbeute: 3,0 g (94% der Theorie) (5RS,6SR)-8-Methoxycarbonyl-8-azabicyclo[4.3.0]non-1-en-5-carbonsäure,
Schmelzpunkt: 93-95°C,
¹H-NMR (CDCl₃): δ 10,5-9,50 (m, 1H), 5,65 (m, 1H), 4,08-3,90 (m, 2H), 3,85-3,70 (m, 1H), 3,72 (s, 3H), 3,39 (t, 1H), 3,07-3,00 (m, 1H), 2,95-2,83 (m, 1H), 2,35-2,24 (m, 1H), 2.13-2,01 (m, 2H), 1,88-1,79 ppm (m, 1H).

### Beispiel Z 17

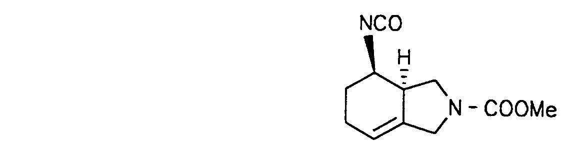

Es werden 3,0 g (0.01 mol) (5RS,6SR)-8-Methoxycarbonyl-8-azabicyclo[4.3.0]non-1-en-5-carbonsäure in 50 ml Toluol vorgelegt und mit 1,4 g Triethylamin und 3,8 g Diphenylphosphorylazid vermischt. Man erwärmt 8 Stunden auf 60°C, gießt die abgekühlte Lösung auf Eiswasser, trennt die organische Phase ab und extrahiert noch mehrfach mit Toluol. Die vereinigten Extrakte werden getrocknet, eingeengt und ohne weitere Reinigung für die folgende Umsetzung verwendet.
Rohausbeute: 5,2 g eines braunen Öls, das als Hauptbestandteil eine Mischung aus (5RS,6SR)- und (5RS,6RS)-8-Methoxycarbonyl-8-azabicyclo[4.3.0]non-1-en-5-ylisocyanat enthält,
IR: 2150 bzw. 2250 cm⁻¹

### Beispiel Z 18

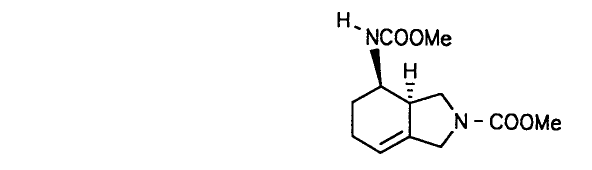

Es werden 5,2 g rohes (5RS,6SR)-8-Methoxycarbonyl-8-azabicyclo[4.3.0]non-1-en-5-isocyanat in 70 ml Methanol gelöst und 15 Stunden zum Rückfluß erhitzt. Die erkaltete Mischung wird eingeengt und an Kieselgel mit einer Mischung aus Cyclohexan und Aceton (2:1) chromatographiert.
Ausbeute: 1,6 g (45 % der Theorie, bezogen auf die Säure) (5RS,6SR)-5-Methoxycarbonylamino-8-azabicyclo[4.3.0]non-1-en-8-carbonsäuremethylester,
Schmelzpunkt: 113-117°C,
¹H-NMR (CDCl₃): δ 5,65 (m, 1H), 4,71 (m, 1H), 4,25 (m, 1H), 4,07-3,70 (m, 3H), 3,69 (s, 3H), 3,65 (s, 3H), 3,50 (m, 1H), 3,08 (dt, 1H), 2,88 (m, 1H), 2,22-2,05 (m, 2H), 2,00-1,87 (m, 1H), 1,75-1,64 ppm (m, 1H).

### Beispiel Z 19

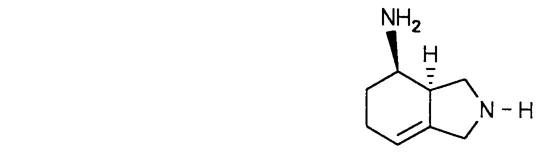

Es werden 1,20 g (0,005 mol) (5RS,6SR)-5-Methoxycarbonylamino-8-azabicyclo[4.3.0]non-1-en-8-carbonsäuremethylester in 5 ml Ethanol gelöst und mit 100 g einer gesättigten Bariumhydroxidlösung versetzt. Die Mischung wird zwei Tage unter Rückfluß erhitzt, abgekühlt, das ausgefallene Bariumcarbonat abfiltriert und das Filtrat mehrfach mit Chloroform extrahiert. Die vereinigten organischen Phasen werden getrocknet und eingeengt.
Ausbeute: 0,60 g (Rohausbeute 92 % der Theorie) eines braunen Öls: (5RS,6SR)-5-Amino-8-azabicyclo[4.3.0]non-1-en [(3aSR,4RS)-2,3,3a,4,5,6-Hexahydro-1H-isoindol-4-ylamin],
¹H-NMR (CDCl₃): δ 5,55 (m, 1H), 3,49 (dd, 2H), 3,32 (m, 1H), 3,13 (m, 1H), 2,75 (t, 1H), 2,47-2,59 (m, 1H), 2,10-1,88 (m, 2H), 1,92 (m, 3H), 1,73-1,53 ppm (m, 2H).

### Herstellung der Wirkstoffe

### Beispiel 1

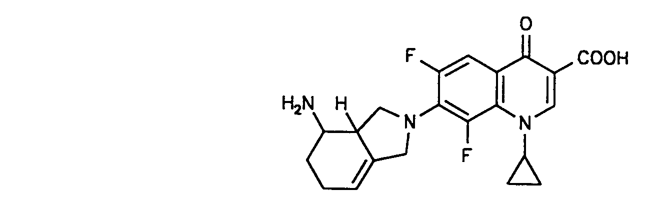

283 mg (1 mmol) 1-Cyclopropyl-6,7,8-trifluor-1,4-dihydro-4-oxo-3-chinolincarbonsäure werden in einer Mischung aus 2 ml Acetonitril und 1 ml Dimethylformamid mit 224 mg (2 mmol) 1,4-Diazabicyclo[2.2.2]octan und 152 mg (1,1 mmol) (3aRS,4RS)-2,3,3a,4,5,6-Hexahydro-1H-isoindol-4-ylamin versetzt und 1 Stunde unter Rückfluß erhitzt. Die Mischung wird im Vakuum eingeengt, der Rückstand mit etwa 40 ml Wasser verrührt und der ausgefallene Rückstand nach dem Trocknen bei 70°C im Hochvakuum chromatographisch gereinigt: Kieselgel, Dichlormethan/Methanol/17 % wäßriges Ammoniak (30:8:1).
Ausbeute: 197 mg (49 % der Theorie) 7-[(3aRS,4RS)-4-Amino-2,3,3a,4,5,6-hexahydro-1H-isoindol-2-yl]-1-cyclopropyl-6,8-difluor-1,4-dihydro-4-oxo-3-chinolincarbonsäure,
Schmelzpunkt: 205-207°C (unter Zersetzung).

### Beispiel 2

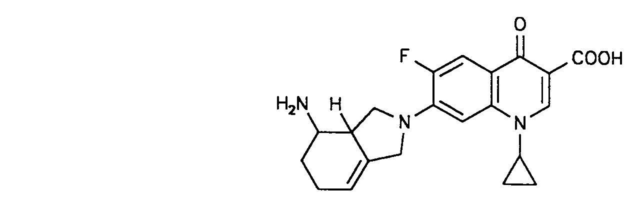

Unter entsprechenden Bedingungen wie in Beispiel 1 erhält man mit 1-Cyclopropyl-6,7-difluor-1,4-dihydro-4-oxo-3-chinolincarbonsäure in 29 %-iger Ausbeute 7-[(3aRS,4RS)-4-Amino-2,3,3a,4,5,6-hexahydro-1H-isoindol-2-yl]-1-cyclopropyl-6-fluor-1,4-dihydro-4-oxo-3-chinolincarbonsäure Schmelzpunkt 210-213°C (unter Zersetzung).

### Beispiel 3

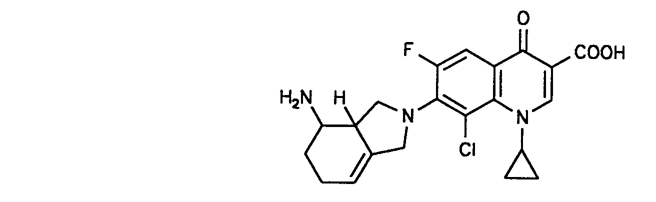

Unter entsprechenden Bedingungen wie in Beispiel 1 erhält man mit 8-Chlor-1-cyclopropyl-6,7-difluor-1,4-dihydro-4-oxo-3-chinolincarbonsäure in 85 %-iger Ausbeute 7-[(3aRS,4RS)-4-Amino-2,3,3a,4,5,6-hexahydro-1H-isoindol-2-yl]-8-chlor-1-cyclopropyl-6-fluor-1,4-dihydro-4-oxo-3-chinolincarbonsäure vom Schmelzpunkt 181-184°C (unter Zersetzung).

### Beispiel 4

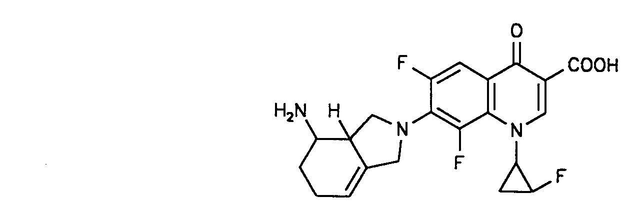

Unter entsprechenden Bedingungen wie in Beispiel 1 erhält man mit 6,7,8-Trifluor-1-(cis-2-fluor-cyclopropyl)-1,4-dihydro-4-oxo-3-chinolincarbonsäure in 68 %-iger Ausbeute 7-[(3aRS,4RS)-4-Amino-2,3,3a,4,5,6-hexahydro-1H-isoindol-2-yl]-6,8-difluor-1-(cis-2-fluor-cyclopropyl)-1,4-dihydro-4-oxo-3-chinolincarbonsäure vom Schmelzpunkt 210-205°C (unter Zersetzung).

### Beispiel 5

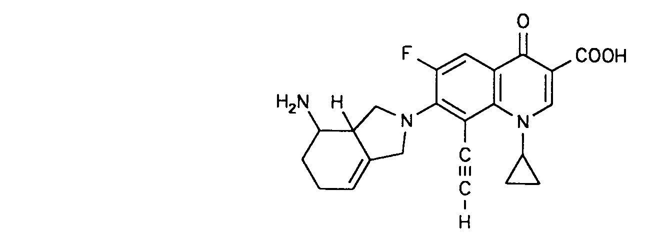

Unter entsprechenden Bedingungen wie in Beispiel 1 erhält man mit 1-Cyclopropyl-8-ethinyl-6,7-difluor-1,4-dihydro-4-oxo-3-chonolincarbonsäure in 77 %-iger Ausbeute 7-[(3aRS,4RS)-4-Amino-2,3,3a,4,5,6-hexahydro-1H-isoindol-2-yl]-1-cyclopropyl-8-ethinyl-6-fluor-1,4-dihydro-4-oxo-3-chinolincarbonsäure vom Schmelzpunkt 270-274°C (unter Zersetzung).

### Beispiel 6

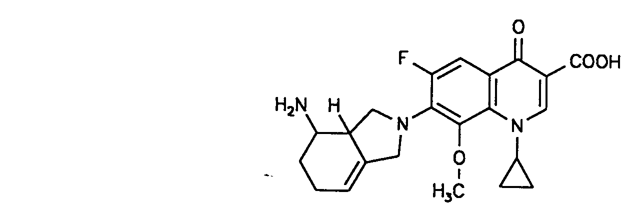

Unter entsprechenden Bedingungen wie in Beispiel 1 erhält man mit 1-Cyclopropyl-6,7-difluor-1,4-dihydro-8-methoxy-4-oxo-3-chinolincarbonsäure in 67 %-iger Ausbeute 7-[(3aRS,4RS)-4-Amino-2,3,3a,4,5,6-hexahydro-1H-isoindol-2-yl]-1-cyclopropyl-6-fluor-1,4-dihydro-8-methoxy-4-oxo-3-chinolincarbonsäure vom Schmelzpunkt 236-238°C (unter Zersetzung).

### Beispiel 7

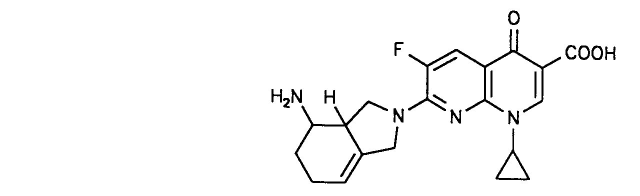

283 mg (1 mmol) 7-Chlor-1-cyclopropyl-6-fluor-1,4-dihydro-4-oxo-1,8-naphthyridin-3-carbonsäure werden in 6 ml Acetonitril mit 332 mg (2,4 mmol) (3aRS,4RS)-2,3,3a,4,5,6-Hexahydro-1H-isoindol-4-ylamin versetzt und 1 Stunde auf 50°C erwärmt. Der ausgefallene Niederschlag wird abgesaugt, mit Acetonitril gewaschen und chromatographisch gereinigt: Kieselgel, Dichlormethan/Methanol/17 % wäßriges Ammoniak (30:8:1).
Ausbeute: 76 mg (20 % der Theorie) 7-[(3aRS,4RS)-4-Amino-2,3,3a,4,5,6-hexahydro-1H-isoindol-2-yl]-1-cyclopropyl-6-fluor-1,4-dihydro-4-oxo-1,8-naphthyridin-3-carbonsäure,
Schmelzpunkt: 263-265°C (unter Zersetzung).

### Beispiel 8

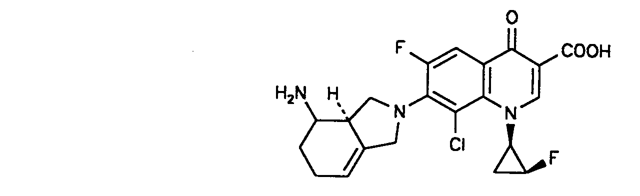

Unter entsprechenden Bedingungen wie in Beispiel 1 erhält man mit 8-Chlor-6,7-difluor-1-[(1R,2S)-2-fluor-cyclopropyl]-1,4-dihydro-4-oxo-3-chinolincarbonsäure und (3aSR,4RS)-2,3,3a,4,5,6-Hexahydro-1H-isoindol-4-ylamin in 84 %-iger Ausbeute 7-[(3aSR,4RS)-4-Amino-2,3,3a,4,5,6-hexahydro-1H-isoindol-2-yl]-8-chlor-6-fluor 1-[(1R,2S)-2-fluor-cyclopropyl]-1,4-dihydro-4-oxo-3-chinolincarbonsäure als Rohprodukt, das chromatographisch an Kieselgel (Laufmittel: Dichlormethan/Methanol/17 %-iges wäßriges Ammoniak 30:8:1) gereinigt wird; Schmelzpunkt: ab 192°C (unter Zersetzung).

### Beispiel 9

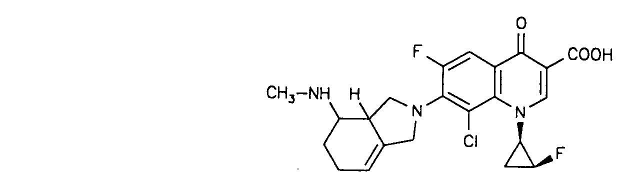

Unter entsprechenden Bedingungen wie in Beispiel 1 erhält man mit 8-Chlor-6,7-difluor-1-[(1R,2S)-2-fluor-cyclopropyl]-1,4-dihydro-4-oxo-3-chinolincarbonsäure und (3aRS,4RS)-2,3,3a,4,5,6-Hexahydro-1H-isoindol-4-yl-methyl-amin in 75 %iger Ausbeute ein Diastereomerengemisch von 8-Chlor-6-fluor-1-[(1R,2S)-2-fluorcyclopropyl]-1,4-dihydro-7-[(3aRS,4RS)-4-methylamino-2,3,3a,4,5,6-hexahydro-1H-isoindol-2-yl]-4-oxo-3-chinolincarbonsäure, das sich durch Chromatographie an Kielselgel auftrennen läßt, wobei man zunächst mit dem Laufmittelsystem Dichlormethan/Methanol (95:5) eluiert und anschließend auf das System Dichlormethan/Methanol/17%-Ammoniak (30:8:1) wechselt. Man erhält die beiden Diastereomeren nach dem Einengen der entsprechenden Fraktionen in 22 % beziehungsweise 25 % der Theorie:
A. R_{f}-Wert: 0,28; Kieselgel, Dichlormethan/Methanol/17%-Ammoniak (30:8:1);
   FAB-Massenspektrum: m/e: 450 [(M+H)⁺].
B. R_{f}-Wert: 0,19; Kieselgel, Dichlormethan/Methanol/17%-Ammoniak (30:8:1);
   FAB-Massenspektrum: m/e: 450 [(M+H)⁺].

### Beispiel 10

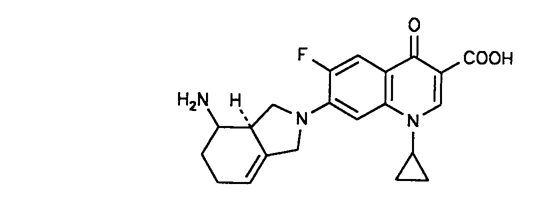

133 mg (0,5 mmol) 1-Cyclopropyl-6,7-difluor-1,4-dihydro-4-oxo-3-chinolincarbonsäure werden in einer Mischung aus 2 ml Acetonitril und 1 ml Dimethylformamid mit 85 mg (0,76 mmol) 1,4-Diazabicyclo[2.2.2]octan und 90 mg (0,65 mmol) (3aSR,4RS)-2,3,3a,4,5,6-Hexahydro-1H-isoindol-4-ylamin versetzt und 1 Stunde unter Rückfluß erhitzt. Die Mischung wird bei 60°C/15 mbar eingeengt, der Rückstand mit etwa 20 ml Wasser im Ultraschallbad behandelt und der ausgefallene Niederschlag abgesaugt, mit Wasser gewaschen und bei 100°C im Vakuum getrocknet.
Ausbeute: 108 mg (56 % der Theorie) 7-[(3aSR,4RS)-4-Amino-2,3,3a,4,5,6-hexahydro-1H-isoindol-2-yl]-1-cyclopropyl-6-fluor-1,4-dihydro-4-oxo-3-chinolincarbonsäure,
Schmelzpunkt: 262-264°C (unter Zersetzung).

## Patentansprüche

1. Verbindungen der Formel (I)
T-Q (I)
in welcher
Q einen Rest der Formeln 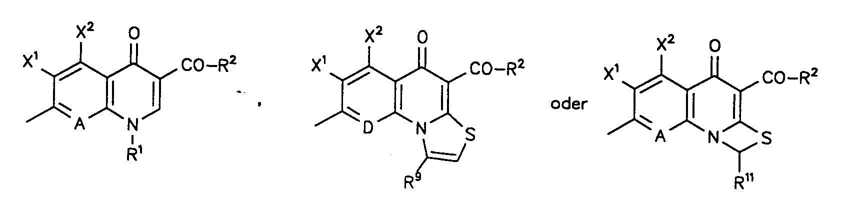 bedeutet, worin
R¹ für gegebenenfalls durch Halogen oder Hydroxy ein- oder zweifach substituiertes Alkyl mit 1 bis 4 Kohlenstoffatomen, Alkenyl mit 2 bis 4 Kohlenstoffatomen, gegebenenfalls durch 1 oder 2 Fluoratome substituiertes Cycloalkyl mit 3 bis 6 Kohlenstoffatomen, Bicyclo[1.1.1]pent-1-yl, 1,1-Dimethylpropargyl, 3-Oxetanyl, Methoxy, Amino, Methylamino, Dimethylamino, gegebenenfalls durch Halogen, Amino oder Hydroxy ein- oder zweifach substituiertes Phenyl,
R² für Hydoxy, gegebenenfalls durch Hydroxy, Methoxy, Amino, Dimethylamino substituiertes Alkoxy mit 1 bis 3 Kohlenstoffatomen, Benzyloxy oder (5-Methyl-2-oxo-1,3-dioxol-4-yl)-methyloxy, Acetoxymethyloxy, Pivaloyloxymethyloxy, 5-Indanyloxy, Phthalidinyloxy, 3-Acetoxy-2-oxo-butyloxy, Nitromethyl oder Dialkoxycarbonylmethyl mit 1 bis 2 Kohlenstoffatomen in jedem Alkylteil,
R⁹ für Wasserstoff oder gegebenenfalls durch Methoxy, Hydroxy oder Halogen substituiertes Alkyl mit 1 bis 3 Kohlenstoffatomen,
R¹¹ für Wasserstoff, CH₃, CH₂F oder =CH₂,
X¹ für Wasserstoff, Halogen oder Nitro,
X² für Wasserstoff, Halogen, Amino, Hydroxy, Methoxy, Mercapto, Methyl, Halogenmethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*S-CH₂-CH-CH₃, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff, Methyl oder Formyl bedeutet, bilden kann, und
D für N oder C-R¹⁰ steht, worin
R¹⁰ für Wasserstoff, Halogen, CF₃, OCH₃, OCHF₂ oder CH₃ steht oder auch gemeinsam mit R⁹ eine Brücke der Struktur -*O-CH₂-, -*NH-CH₂-, -*N(CH₃)-CH₂-, -*N(C₂H₅)-CH₂-, -*N(C₃H₅)-CH₂- oder -*S-CH₂- bilden kann, wobei das mit * markierte Atom mit dem Kohlenstoffatom von D verknüpft ist, und
T einen Rest der Formel 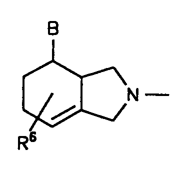
bedeutet, worin
B für (CH₂)ₘ-NR³R⁴ oder (CH₂)ₘ-OR⁵ steht, worin
m für 0 oder 1,
R³ für Wasserstoff, gegebenenfalls durch Hydroxy substituiertes Alkyl mit 1 bis 3 Kohlenstoffatomen, Acyl mit 1 bis 3 Kohlenstoffatomen oder Alkoxycarbonyl mit 1 bis 4 Kohlenstoffatomen im Alkylteil,
R⁴ für Wasserstoff oder Methyl und
R⁵ für Wasserstoff oder Methyl und
R⁶ für Wasserstoff oder Methyl steht,
und deren pharmazeutisch verwendbaren Hydrate und Säureadditionssalze sowie die Alkali-, Erdalkali-, Silber- und Guanidiniumsalze der zugrundeliegenden Carbonsäuren.

2. Verbindungen der Formel (I) gemäß Anspruch 1,
in welcher
Q einen Rest der Formeln 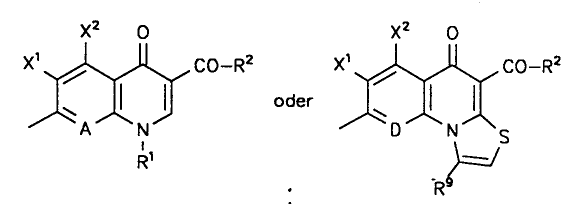
bedeutet, worin
R¹ für gegebenenfalls durch Halogen ein- oder zweifach substituiertes Alkyl mit 1 bis 4 Kohlenstoffatomen, Alkenyl mit 2 bis 3 Kohlenstoffatomen, gegebenenfalls durch 1 Fluoratom substituiertes Cycloalkyl mit 3 oder 4 Kohlenstoffatomen, Bicyclo[1.1.1]pent-1-yl, 1,1-Dimethylpropargyl, 3-Oxetanyl, Methylamino, gegebenenfalls durch Fluor, Amino oder Hydroxy ein- oder zweifach substituiertes Phenyl,
R² für Hydroxy, Alkoxy mit 1 bis 2 Kohlenstoffatomen, Benzyloxy oder (5-Methyl-2-oxo-1,3-dioxol-4-yl)-methyloxy,
R⁹ für Wasserstoff oder gegebenenfalls durch Fluor ein- bis dreifach substituiertes Alkyl mit 1 bis 2 Kohlenstoffatomen,
X¹ für Fluor oder Chlor,
X² für Wasserstoff, Halogen, Amino, Methyl, Trifluormethyl oder Vinyl,
A für N oder C-R⁷ steht, worin
R⁷ für Wasserstoff, Halogen, CF₃, OCH₃, OCHF₂, CH₃, CN, CH=CH₂ oder C≡CH steht oder auch gemeinsam mit R¹ eine Brücke der Struktur -*O-CH₂-CH-CH₃, -*S-CH₂-CH₂-, -*CH₂-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet, bilden kann, und
D für N oder C-R¹⁰ steht, worin
R¹⁰ für Wasserstoff, Fluor, Chlor, CF₃, OCH₃ oder CH₃ steht oder auch gemeinsam mit R⁹ eine Brücke der Struktur -O-CH₂-, -*N(CH₃)-CH₂-, -*N(C₂H₅)-CH₂, -*N(C₃H₅)-CH₂- oder -*S-CH₂- bilden kann, wobei das mit * markierte Atom mit dem Kohlenstoffatom von D verknüpft ist, und
T einen Rest der Formel 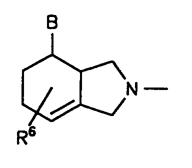
bedeutet, worin
B für -NR³R⁴ oder -OH steht, worin
R³ für Wasserstoff oder Methyl,
R⁴ für Wasserstoff oder Methyl und
R⁶ für Wasserstoff steht
und deren pharmazeutisch verwendbaren Hydrate und Säureadditionssalze sowie die Alkali-, Erdalkali-, Silber- und Guanidiniumsalze der zugrundeliegenden Carbonsäuren.

3. Verbindungen der Formel (I) gemäß Anspruch 1,
in welcher
Q einen Rest der Formel 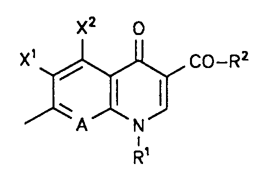 bedeutet, worin
R¹ für gegebenenfalls durch Fluor ein- oder zweifach substituiertes Alkyl mit 1 bis 4 Kohlenstoffatomen, Vinyl, gegebenenfalls durch 1 Fluoratom substituiertes Cyclopropyl, gegebenenfalls durch Fluor ein- oder zweifach substituiertes Phenyl,
R² für Hydroxy, Alkoxy mit 1 bis 2 Kohlenstoffatomen oder (5-Methyl-2-oxo-1,3-dioxol-4-yl)-methyloxy,
X¹ für Fluor,
X² für Wasserstoff, Fluor, Amino, Methyl oder Vinyl,
A für N oder C-R⁷ steht, worin
R⁷ für Wasserstoff, Fluor, Chlor, Brom, CF₃, OCH₃, OCHF₂, CH₃, CN, CH=CH₂ oder C≡CH steht oder auch gemeinsam mit R¹ eine Brücke der Struktur -*O-CH₂-CH-CH₃ oder -*O-CH₂-N-R⁸, wobei das mit * markierte Atom mit dem Kohlenstoffatom von A verknüpft ist und worin
R⁸ Wasserstoff oder Methyl bedeutet,
bilden kann, und
T einen Rest der Formel 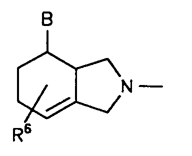
bedeutet, worin
B für NH₂ und
R⁶ für Wasserstoff steht,
und deren pharmazeutisch verwendbaren Hydrate und Säureadditionssalze sowie die Alkali-, Erdalkali-, Silber- und Guanidiniumsalze der zugrundeliegenden Carbonsäuren.

4. Diastereomerenreine und enantiomerenreine Verbindungen nach den Ansprüchen 1 bis 3.

5. Verfahren zur Herstellung von Verbindungen der Formel (I) gemaß Ansprüchen 1 bis 4, dadurch gekennzeichnet, daß man Verbindungen der Formel (II)
Y-Q (II) ,
in welcher
Q die oben angegebene Bedeutung hat und
Y für Halogen, inbesondere für Fluor oder Chlor steht,
mit Verbindungen der Formel (III) 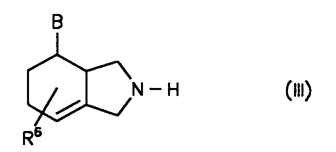 in welcher
B und R⁶ die oben angegebenen Bedeutungen haben,
gegebenenfalls in Gegenwart von Säurefängern umsetzt und gegebenenfalls vorhandene Schutzgruppen abspaltet.

6. Racemische, diastereomerenreine und enantiomerenreine Verbindungen aus der Gruppe bestehend aus
2,3,3a,4,5,6-Hexahydro-1H-isoindol-4-ylamin,
4-Methyl-2,3,3a,4,5,6-hexahydro-1H-isoindol-4-ylamin,
5-Methyl-2,3,3a,4,5,6-hexahydro-1H-isoindol-4-ylamin,
6-Methyl-2,3,3a,4,5,6-hexahydro-1H-isoindol-4-ylamin,
7-Methyl-2,3,3a,4,5,6-hexahydro-1H-isoindol-4-ylamin,
4-Methylamino-2,3,3a,4,5,6-hexahydro-1H-isoindol,
4-Dimethylamino-2,3,3a,4,5,6-hexahydro-1H-isoindol,
4-Aminomethyl-2,3,3a,4,5,6-hexahydro-1H-isoindol,
4-Methylaminomethyl-2,3,3a,4,5,6-hexahydro-1H-isoindol,
4-Hydroxy-2,3,3a,4,5,6-hexahydro-1H-isoindol,
4-Hydroxymethyl-2,3,3a,4,5,6-hexahydro-1H-isoindol.

7. Verbindungen gemäß Ansprüchen 1 bis 4 zur Bekämpfung von Krankheiten.

8. Verbindungen gemäß Ansprüchen 1 bis 4 zur Bekämpfung von bakteriellen Infektionen.

9. Verwendung von Verbindungen gemäß Ansprüchen 1 bis 4 bei der Herstellung von Arzneimitteln.

10. Arzneimittel enthaltend die Verbindungen gemäß Ansprüchen 1 bis 4.

11. Antibakterielle Mittel enthaltend Verbindungen gemäß Ansprüchen 1 bis 4.

## Claims

1. Compounds of the formula (I)
T-Q (I)
in which
Q denotes a radical of the formulae 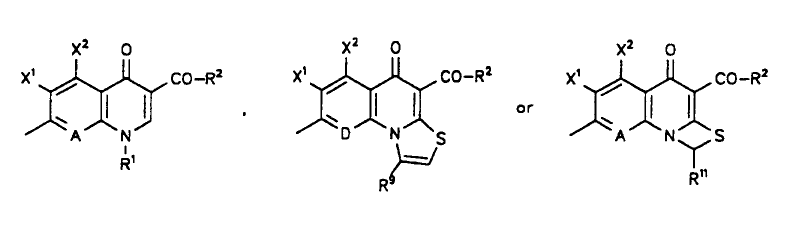 wherein
R¹ represents alkyl which has 1 to 4 carbon atoms and is optionally mono- or disubstituted by halogen or hydroxyl, alkenyl having 2 to 4 carbon atoms, cycloalkyl which has 3 to 6 carbon atoms and is optionally substituted by 1 or 2 fluorine atoms, bicyclo[1.1.1]pent-1-yl, 1,1-dimethylpropargyl, 3-oxetanyl, methoxy, amino, methylamino, dimethylamino or phenyl which is optionally mono- or disubstituted by halogen, amino or hydroxyl,
R² represents hydroxyl, alkoxy which has 1 to 3 carbon atoms and is optionally substituted by hydroxyl, methoxy, amino or dimethylamino, benzyloxy or (5-methyl-2-oxo-1,3-dioxol-4-yl)methyloxy, acetoxymethyloxy, pivaloyloxymethyloxy, 5-indanyloxy, phthalidinyloxy, 3-acetoxy-2-oxo-butyloxy, nitromethyl or dialkoxycarbonylmethyl having 1 to 2 carbon atoms in each alkyl part,
R⁹ represents hydrogen or alkyl which has 1 to 3 carbon atoms and is optionally substituted by methoxy, hydroxyl or halogen,
R¹¹ represents hydrogen, CH₃, CH₂F or =CH₂,
X¹ represents hydrogen, halogen or nitro,
X² represents hydrogen, halogen, amino, hydroxyl, methoxy, mercapto, methyl, halogenomethyl or vinyl,
A represents N or C-R⁷, wherein
R⁷ represents hydrogen, halogen, CF₃, OCH₃, OCHF₂, CH₃, CN, CH=CH₂ or C≡CH, or together with R¹ can also form a bridge having the structure -*O-CH₂-CH-CH₃, -*S-CH₂-CH₂-, -*S-CH₂-CH-CH₃, -*CH₂-CH₂-CH-CH₃ or -*O-CH₂-N-R⁸, wherein the atom labelled with * is linked to the carbon atom of A and wherein
R⁸ denotes hydrogen, methyl or formyl,
and
D represents N or C-R¹⁰_{,} wherein
R¹⁰ represents hydrogen, halogen, CF₃, OCH₃, OCHF₂ or CH₃, or together with R⁹ can also form a bridge having the structure -*O-CH₂-, -*NH-CH₂-, -*N(CH₃)-CH₂-, -*N(C₂H₅)-CH₂-, -*N(C₃H₅)-CH₂- or -*S-CH₂-, wherein the atom labelled with * is linked to the carbon atom of D, and
T denotes a radical of the formula 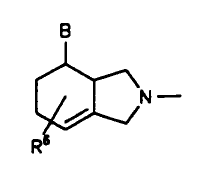 wherein
B represents (CH₂)ₘ-NR³R⁴ or (CH₂)ₘ-OR⁵, wherein
m represents 0 or 1,
R³ represents hydrogen, alkyl which has 1 to 3 carbon atoms and is optionally substituted by hydroxyl, acyl having 1 to 3 carbon atoms or alkoxycarbonyl having 1 to 4 carbon atoms in the alkyl part,
R⁴ represents hydrogen or methyl and
R⁵ represents hydrogen or methyl and
R⁶ represents hydrogen or methyl,
and pharmaceutically usable hydrates and acid addition salts thereof, as well as the alkali metal, alkaline earth metal, silver and guanidinium salts of the underlying carboxylic acids.

2. Compounds of the formula (I) according to Claim 1,
in which
Q denotes a radical of the formulae 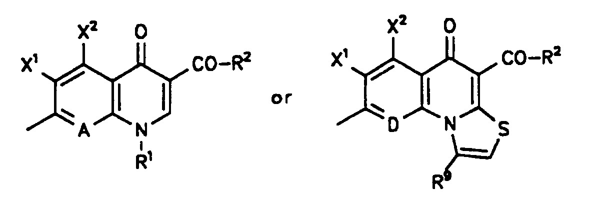
wherein
R¹ represents alkyl which has 1 to 4 carbon atoms and is optionally mono- or disubstituted by halogen, alkenyl having 2 to 3 carbon atoms, cycloalkyl which has 3 or 4 carbon atoms and is optionally substituted by 1 fluorine atom, bicyclo[1.1.1]pent-1-yl, 1,1-dimethylpropargyl, 3-oxetanyl, methylamino or phenyl which is optionally mono- or disubstituted by fluorine, amino or hydroxyl,
R² represents hydroxyl, alkoxy having 1 to 2 carbon atoms, benzyloxy or (5-methyl-2-oxo-1,3-dioxol-4-yl)-methyloxy,
R⁹ represents hydrogen or alkyl which has 1 to 2 carbon atoms and is optionally mono- to trisubstituted by fluorine,
X¹ represents fluorine or chlorine,
X² represents hydrogen, halogen, amino, methyl, trifluoromethyl or vinyl,
A represents N or C-R⁷, wherein
R⁷ represents hydrogen, halogen, CF₃, OCH₃, OCHF₂, CH₃, CN, CH=CH₂ or C≡CH, or together with R¹ can also form a bridge having the structure -*O-CH₂-CH-CH₃, -*S-CH₂-CH₂-, -*CH₂-CH₂-CH-CH₃ or -*O-CH₂-N-R⁸, wherein the atom labelled with * is linked to the carbon atom of A, and wherein
R⁸ denotes hydrogen or methyl,
and
D represents N or C-R¹⁰, wherein
R¹⁰ represents hydrogen, fluorine, chlorine, CF₃, OCH₃ or CH₃, or together with R⁹ can also form a bridge having the structure -O-CH₂-, -*N(CH₃)-CH₂-, -*N(C₂H₅)-CH₂-, -*N(C₃H₅)-CH₂- or -*S-CH₂-, wherein the atom labelled with * is linked to the carbon atom of D, and
T denotes a radical of the formula 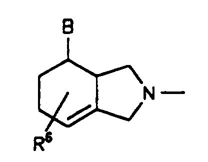
wherein
B represents -NR³R⁴ or -OH, wherein
R³ represents hydrogen or methyl,
R⁴ represents hydrogen or methyl and
and pharmaceutically usable hydrates and acid addition salts thereof and the alkali metal, alkaline earth metal, silver and guanidinium salts of the underlying carboxylic acids.

3. Compounds of the formula (I) according to Claim 1,
in which
Q denotes a radical of the formula 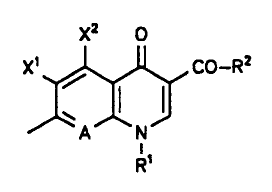
wherein
R¹ represents alkyl which has 1 to 4 carbon atoms and is optionally mono- or disubstituted by fluorine, vinyl, cyclopropyl which is optionally substituted by 1 fluorine atom or phenyl which is optionally mono- or disubstituted by fluorine,
R² represents hydroxyl, alkoxy having 1 to 2 carbon atoms or (5-methyl-2-oxo-1,3-dioxol-4-yl)-methyloxy,
X¹ represents fluorine,
X² represents hydrogen, fluorine, amino, methyl or vinyl,
A represents N or C-R⁷, wherein
R⁷ represents hydrogen, fluorine, chlorine, bromine, CF₃, OCH₃, OCHF₂, CH₃, CN, CH=CH₂ or C≡CH, or together with R¹ can also form a bridge having the structure -*O-CH₂-CH-CH₃ or -*O-CH₂-N-R⁸, where the atom labelled with * is linked to the carbon atom of A, and wherein
R⁸ denotes hydrogen or methyl,
and
T denotes a radical of the formula 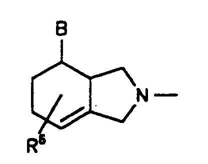
wherein
B represents NH₂ and
R⁶ represents hydrogen,
and pharmaceutically usable hydrates and acid addition salts thereof and the alkali metal, alkaline earth metal, silver and guanidinium salts of the underlying carboxylic acids.

4. Diastereomerically pure and enantiomerically pure compounds according to Claims 1 to 3.

5. Process for the preparation of compounds of the formula (I) according to Claims 1 to 4, characterized in that compounds of the formula (II)
Y-Q (II)
in which
Q has the abovementioned meaning and
Y represents halogen, in particular fluorine or chlorine,
are reacted with compounds of the formula (III) 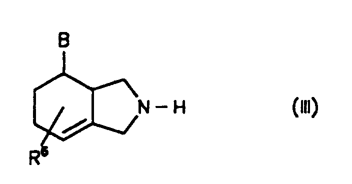 in which
B and R⁶ have the abovementioned meanings,
if appropriate in the presence of acid-trapping agents, and any protective groups are split off.

6. Racemic, diastereomerically pure and enantiomerically pure compounds from the group consisting of
2,3,3a,4,5,6-hexahydro-1H-isoindol-4-ylamine,
4-methyl-2,3,3a,4,5,6-hexahydro-1H-isoindol-4-ylamine,
5-methyl-2,3,3a,4,5,6-hexahydro-1H-isoindol-4-ylamine,
6-methyl-2,3,3a,4,5,6-hexahydro-1H-isoindol-4-ylamine,
7-methyl-2,3,3a,4,5,6-hexahydro-1H-isoindol-4-ylamine,
4-methylamino-2,3,3a,4,5,6-hexahydro-1H-isoindole,
4-dimethylamino-2,3,3a,4,5,6-hexahydro-1H-isoindole,
4-aminomethyl-2,3,3a,4,5,6-hexahydro-1H-isoindole,
4-methylaminomethyl-2,3,3a,4,5,6-hexahydro-1H-isoindole,
4-hydroxy-2,3,3a,4,5,6-hexahydro-1H-isoindole,
4-hydroxymethyl-2,3,3a,4,5,6-hexahydro-1H-isoindole.

7. Compounds according to Claims 1 to 4 for combating diseases.

8. Compounds according to Claims 1 to 4 for combating bacterial infections.

9. Use of compounds according to Claims 1 to 4 in the preparation of medicaments.

10. Medicaments comprising the compounds according to Claims 1 to 4.

11. Antibacterial compositions comprising compounds according to Claims 1 to 4.

## Revendications

1. Composés de formule (I)
T-Q (I)
dans laquelle
Q représente un reste des formules 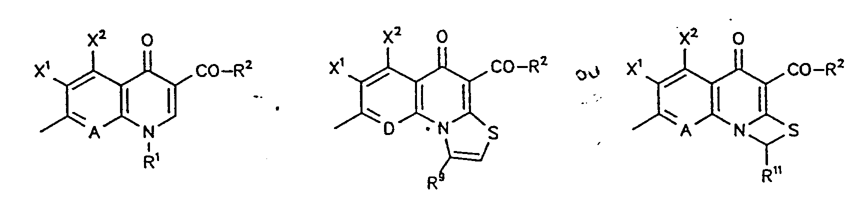 où
R¹ représente un groupe alkyle ayant 1 à 4 atomes de carbone substitué, le cas échéant 1 ou 2 fois par un halogène ou un reste hydroxy, un groupe alcényle ayant 2 à 4 atomes de carbone, un groupe cycloalkyle ayant 3 à 6 atomes de carbone éventuellement substitué par 1 ou 2 atomes de fluor, un groupe bicyclo[1.1.1]pent-1-yle, 1,1-diméthylpropargyle, 3-oxétanyle, méthoxy, amino, méthylamino, diméthylamino, un groupe phényle éventuellement substitué 1 ou 2 fois par un halogène, un reste amino ou hydroxy,
R² représente un groupe hydroxy, un groupe alkoxy ayant 1 à 3 atomes de carbone éventuellement substitué par un reste hydroxy, méthoxy, amino, diméthylamino, un groupe benzyloxy ou un groupe (5-méthyl-2-oxo-1,3-dioxol-4-yl)-méthyloxy, acétoxyxméthyloxy, pivaloyloxyméthyloxy, 5-indanyloxy, phtalidinyloxy, 3-acétoxy-2-oxo-butyloxy, nitrométhyle ou un groupe dialkoxycarbonylméthyle ayant 1 ou 2 atomes de carbone dans chaque partie alkyle,
R⁹ représente de l'hydrogène ou un groupe alkyle ayant 1 à 3 atomes de carbone éventuellement substitué par un reste méthoxy, hydroxy ou halogéno,
R¹¹ est de l'hydrogène, un groupe CH₃, CH₂F ou =CH₂,
X¹ représente de l'hydrogène, un halogène ou un groupe nitro,
X² est de l'hydrogène, un halogène, un groupe amino, hydroxy, méthoxy, mercapto, méthyle, halogénométhyle ou vinyle,
A représente N ou un groupe C-R⁷, dans lequel
R⁷ est de l'hydrogène, un halogène, un groupe CF₃, OCH₃, OCHF₂, CH₃, CN, CH=CH₂ ou C≡CH ou peut aussi former conjointement avec R¹ un pont de structure -*O-CH₂-CH-CH₃, -*S-CH₂-CH₂-, -*S-CH₂-CH₂-CH-CH₃, -*CH₂-CH₂-CH-CH₃ ou -*O-CH₂-N-R⁸, où l'atome marqué d'un astéristique est lié à l'atome de carbone de A et où
R⁸ représente de l'hydrogène, un groupe méthyle ou formyle, et
D représente N ou un groupe C-R¹⁰, dans lequel
R¹⁰ est de l'hydrogène, un halogène, un radical CF₃, OCH₃, OCHF₂ ou CH₃ ou peut aussi former conjointement avec R⁹ un pont de structure -*O-CH₂-, -*NH-CH₂-, -*N(CH₃)-CH₂-, -*N(C₂H₅)-CH₂-, -*N(C₃H₅)-CH₂- ou -*S-CH₂-, où l'atome marqué d'un astérisque est lié à l'atome de carbone de D, et
T représente un reste de formule 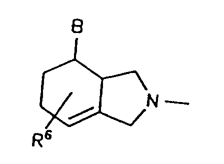
dans laquelle
B est un groupe (CH₂)ₘ-NR³R⁴ ou (CH₂)ₘ-OR⁵, dans lequel
m a la valeur 0 ou 1,
R³ est de l'hydrogène, un groupe alkyle ayant 1 à 3 atomes de carbone éventuellement substitué par un reste hydroxy, un groupe acyle ayant 1 à 3 atomes de carbone ou un groupe alkoxycarbonyle ayant 1 à 4 atomes de carbone dans la partie alkyle,
R⁴ est le l'hydrogène ou un groupe méthyle et
R⁵ est de l'hydrogène ou un groupe méthyle, et
R⁶ est de l'hydrogène ou un groupe méthyle,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

2. Composés de formule (I) suivant la revendication 1, dans laquelle
Q est un reste des formules 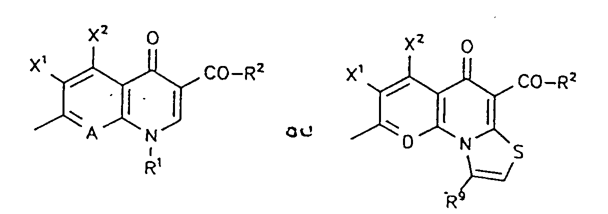
où
R¹ représente un groupe alkyle ayant 1 à 4 atomes de carbone éventuellement substitué 1 ou 2 fois par un halogène, un groupe alcényle ayant 2 ou 3 atomes de carbone, un groupe cycloalkyle ayant 3 ou 4 atomes de carbone éventuellement substitué par un atome de fluor, un groupe bicyclo[1.1.1]pent-1-yle, 1,1-diméthylpropargyle, 3-oxétanyle, méthylamino, un groupe phényle éventuellement substitué 1 ou 2 fois par du fluor, un reste amino ou hydroxy,
R² est un groupe hydroxy, un groupe alkoxy ayant 1 ou 2 atomes de carbone, un groupe benzyloxy ou un groupe (5-méthyl-2-oxo-1,3-dioxol-4-yl)-méthyloxy,
R⁹ est de l'hydrogène ou un groupe alkyle ayant 1 ou 2 atomes de carbone éventuellement substitué 1 à 3 fois par du fluor,
X¹ représente du fluor ou du chlore,
X² est de l'hydrogène, un halogène, un groupe amino, méthyle, trifluorométhyle ou vinyle,
A représente N ou un groupe C-R⁷, dans lequel
R⁷ est de l'hydrogène, un halogène, un groupe CF₃, OCH₃, OCHF₂, CH₃, CN, CH=CH₂ ou C≡CH ou peut aussi former conjointement avec R¹ un pont de structure -*O-CH₂-CH-CH₃, -*S-CH₂-CH₂-, -*CH₂-CH₂-CH-CH₃ ou -*O-CH₂-N-R⁸, où l'atome marqué d'un astéristique est lié à l'atome de carbone de A et où
R⁸ représente de l'hydrogène ou un groupe méthyle, et
D représente N ou un groupe C-R¹⁰, dans lequel
R¹⁰ est de l'hydrogène, du fluor, du chlore, un radical CF₃, OCH₃, ou CH₃ ou peut aussi former conjointement avec R⁹ un pont de structure -*O-CH₂-, -*N(CH₃)-CH₂-, -*N(C₂H₅)-CH₂-, -*N(C₃H₅)-CH₂- ou -*S-CH₂-, où l'atome marqué d'un astérisque est lié à l'atome de carbone de D, et
T est un reste de formule 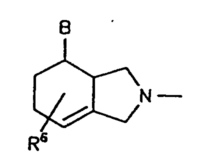
dans laquelle
B est un groupe -NR³R⁴ ou -OH, où
R³ est de l'hydrogène ou un radical méthyle,
R⁴ est le l'hydrogène ou un radical méthyle et
R⁶ est de l'hydrogène,
et leurs hydrates et leurs sels d'addition d'acides acceptables du point de vue pharmaceutique ainsi que les sels de métaux alcalins, de métaux alcalino-terreux, d'argent et de guanidinium, des acides carboxyliques de base.

3. Composés de formule (I) suivant la revendication 1, dans laquelle
Q représente un reste de formule 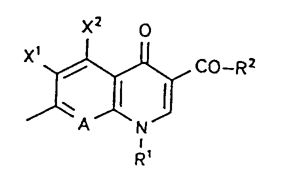
où
R¹ est un groupe alkyle ayant 1 à 4 atomes de carbone éventuellement substitué 1 ou 2 fois par du fluor, un groupe vinyle, un groupe cyclopropyle éventuellement substitué par un atome de fluor, un groupe phényle éventuellement substitué 1 ou 2 fois par du fluor,
R² est un groupe hydroxy, alkoxy ayant 1 ou 2 atomes de carbone ou (5-méthyl-2-oxo-1,3-dioxol-4-yl)-méthyloxy,
X¹ est du fluor,
X² est de l'hydrogène, du fluor, un groupe amino, méthyle ou vinyle,
A représente N ou un groupe C-R⁷, dans lequel
R⁷ est de l'hydrogène, du fluor, du chlore, un radical CF₃, OCH₃, OCHF₂, CH₃, CN, CH=CH₂ ou C≡CH ou peut aussi former conjointement avec R¹ un pont de structure -*O-CH₂-CH-CH₃ ou -*O-CH₂-N-R⁸, où l'atome marqué d'un astérisque est lié à l'atome de carbone de A et où
R⁸ est de l'hydrogène ou un radical méthyle, et
T représente un reste de formule 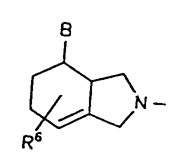 dans laquelle
B est un groupe NH₂ et
R⁶ est de l'hydrogène,
et leurs hydrates et leurs sels d'addition d'acides acceptables du point de vue pharmaceutique ainsi que les sels de métaux alcalins, de métaux alcalino-terreux, d'argent et de guanidinium des acides carboxyliques de base.

4. Composés diastéréo-isomères purs et énantiomères purs suivant les revendications 1 à 3.

5. Procédé de production de composés de formule (I) suivant les revendications 1 à 4, caractérisé en ce qu'on fait réagir des composés de formule (II)
Y-Q (II)
dans laquelle
Q a la définition indiquée ci-dessus et
Y représente un halogène, en particulier le fluor ou le chlore,
avec des composés de formule (III) 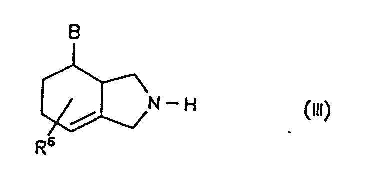 dans laquelle
B et R⁶ ont les définitions indiquées ci-dessus,
le cas échéant, en présence d'accepteurs d'acides et on élimine les groupes protecteurs éventuellement présents.

6. Composés racémiques, diastéréo-isomères purs et énantiomères purs du groupe consistant en
la 2,3,3a,4,5,6-hexahydro-1H-iso-indole-4-ylamine,
la 4-méthyl-2,3,3a,4,5,6-hexahydro-1H-iso-indole-4-ylamine,
la 5-méthyl-2,3,3a,4,5,6-hexahydro-1H-iso-indole-4-ylamine,
la 6-méthyl-2,3,3a,4,5,6-hexahydro-1H-iso-indole-4-ylamine,
la 7-méthyl-2,3,3a,4,5,6-hexahydro-1H-iso-indole-4-ylamine,
le 4-méthylamino-2,3,3a,4,5,6-hexahydro-1H-iso-indole,
le 4-diméthylamino-2,3,3a,4,5,6-hexahydro-1H-iso-indole,
le 4-aminométhyl-2,3,3a,4,5,6-hexahydro-1H-iso-indole,
le 4-méthylaminométhyl-2,3,3a,4,5,6-hexahydro-1H-iso-indole,
le 4-hydroxy-2,3,3a,4,5,6-hexahydro-1H-iso-indole,
le 4-hydroxyméthyl-2,3,3a,4,5,6-hexahydro-1H-iso-indole.

7. Composés suivant les revendications 1 à 4, destinés à combattre des maladies.

8. Composés suivant les revendications 1 à 4, destinés à combattre des infections bactériennes.

9. Utilisation de composés suivant les revendications 1 à 4, dans la préparation de médicaments.

10. Médicaments contenant les composés suivant les revendications 1 à 4.

11. Compositions antibactériennes contenant des composés suivant les revendications 1 à 4.
